# EUROPEAN PATENT APPLICATION

(11) **EP 4 834 306 A1**
(43) Date of publication of application: **04.11.2026**
(21) Application number: 24913015.4
(22) Date of filing: 26.12.2024
(51) Int. Cl.: A61K 6/831, A61K 6/836, A61K 6/887

(54) **TWO-COMPONENT DENTAL COMPOSITION**

(30) Priority: 28.12.2023 JP 2023223470
(71) Applicant: Kuraray Noritake Dental Inc., Kurashiki-shi, Okayama 710-0801 (JP)
(72) Inventor: MURAYAMA Momoe, Tainai-shi, Niigata 959-2653 (JP); KAWANA Mariko, Tainai-shi, Niigata 959-2653 (JP)
(74) Representative: D Young & Co LLP
(86) International application number: PCT/JP2024/046170
(87) International publication number: WO 2025/143129

(57) **Abstract**

The present invention provides a two-pack type dental composition that undergoes little color change and exhibits excellent color stability over extended periods. The present invention relates to a two-pack type dental composition comprising an ultraviolet absorber (A), a polymerizable monomer (B), a chemical polymerization initiator (C), and a chemical polymerization accelerator (D), wherein the ultraviolet absorber (A) is a triazine-based ultraviolet absorber having a maximum absorption wavelength in a wavelength range of 310 nm or more and 400 nm or less. The ultraviolet absorber (A) is preferably a triazine-based ultraviolet absorber represented by general formula (1).

## Description

### TECHNICAL FIELD

The present invention relates to a two-pack type dental composition. More specifically, the invention relates to a two-pack type dental composition that undergoes little color change and exhibits long-term color stability.

### BACKGROUND ART

Adhesive materials are used for restorative treatment of moist materials, for example, biological hard tissues such as tooth structure and bones. Generally, resin-based dental curable compositions containing components such as radical polymerizable monomers and polymerization initiators are used as adhesive materials for such moist materials.

Teeth that have lost their function due to causes such as caries or accidents are restored by cementing, for example, metal or ceramic crown restoration materials, such as inlays or crowns, to the tooth. An adhesive known as a dental cement is used to cement such crown restoration materials to the tooth.

When the defect is small in the restoration of tooth structure damaged by defects such as caries, the mainstream method of restoration typically involves filling the detect with a paste of a restoration material called a filling composite resin, mainly composed of a (meth)acrylate radical polymerizable monomer and an inorganic filler such as a metal oxide, followed by forming the paste into the shape of the tooth, and polymerizing and curing of the material.

The method of polymerization employed in dentistry is broadly classified into photopolymerization and chemical polymerization.

A photopolymerizable restoration material using a radical polymerizable monomer and a photopolymerization initiator hardly undergoes polymerization reaction in the absence of light, offering various advantages such as allowing all components to be produced and stored in a single-paste form, eliminating the mixing and kneading procedure required for chemically polymerizable restoration materials at the time of use, and providing a long working time. This has made photopolymerizable dental restoration materials mainstream in recent years. In the following, such photopolymerizable restoration materials are referred to as dental photopolymerizable compositions.

In the restoration of tooth structure using dental photopolymerizable compositions, it is important for favorable aesthetics that the restored portion have the same color tone as the surrounding tooth structure. However, the polymerized and cured product of the dental photopolymerizable composition undergoes substantial discoloration in a relatively short time period when exposed to ultraviolet light from sunlight or other light sources.

Discoloration in the cured polymerization product causes a shift from the color tone adjusted to match the surrounding tooth structure at the time of restoration, resulting in loss of the initial aesthetics and failure to satisfy the requirements for dental treatment. For example, JIS T 6514:2015 specifies that discoloration of the cured product remain not readily detectable by the naked eye even after 10 hours of sunlight exposure.

An ultraviolet absorber is incorporated in the dental photopolymerizable composition to prevent discoloration of the cured product due to ultraviolet light from sunlight or other light sources.

Known examples of such ultraviolet absorbers incorporated in the dental photopolymerizable composition include benzophenone-based ultraviolet absorbers, benzotriazole-based ultraviolet absorbers, and triazine-based ultraviolet absorbers. Among these, benzotriazole-based ultraviolet absorbers are known to provide excellent stability to sunlight (for example, see Patent Literatures 1 and 2).

### CITATION LIST

### Patent Literature

Patent Literature 1: JP2004-231913 A
Patent Literature 2: JP2006-117543 A
Patent Literature 3: JP2016-166138 A

### SUMMARY OF INVENTION

### Technical Problem

However, the ultraviolet absorbers of Patent Literatures 1 to 3 were found to involve certain problems. During prolonged storage, the ultraviolet absorbers may cause coloration by forming quinoid structures or complexes with components such as chemical polymerization initiators and chemical polymerization accelerators contained in the dental curable composition, or may react with these components and affect curability.

It is an object of the present invention to provide a two-pack type dental composition that undergoes little color change and exhibits excellent color stability over extended periods.

### Solution to Problem

The present inventors conducted intensive studies, and found that the foregoing issues can be overcome with a specific two-pack type dental composition that comprises an ultraviolet absorber (A), a polymerizable monomer (B), a chemical polymerization initiator (C), and a chemical polymerization accelerator (D), and in which the ultraviolet absorber (A) is a triazine-based ultraviolet absorber having a maximum absorption wavelength in a wavelength range of 310 nm or more and 400 nm or less. This led to the completion of the present invention.

Specifically, the present invention includes the following.
[1] A two-pack type dental composition comprising an ultraviolet absorber (A), a polymerizable monomer (B), a chemical polymerization initiator (C), and a chemical polymerization accelerator (D),
   wherein the ultraviolet absorber (A) is a triazine-based ultraviolet absorber having a maximum absorption wavelength in a wavelength range of 310 nm or more and 400 nm or less.
[2] The two-pack type dental composition according to [1], wherein the ultraviolet absorber (A) is a triazine-based ultraviolet absorber represented by the following general formula (1), 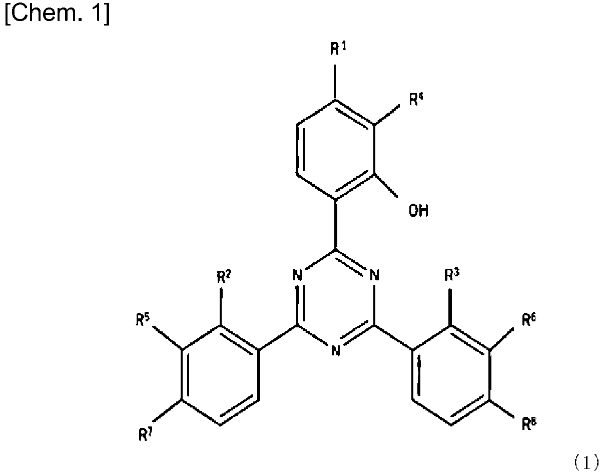 wherein R¹ represents an alkyl group or an alkoxy group, R² and R³ are the same or different from each other and each represent a hydrogen atom, a hydroxyl group, an alkyl group, an alkoxy group, or a halogen atom, R⁴, R⁵, and R⁶ are the same or different from each other and each represent a hydrogen atom or an alkyl group, and R⁷ and R⁸ are the same or different from each other and each represent a hydrogen atom, a hydroxyl group, an alkyl group, an alkoxy group, an aryl group, or a halogen atom, where the alkyl groups and alkoxy groups of R¹ to R⁸ may be optionally substituted.
[3] The two-pack type dental composition according to [1] or [2], wherein the ultraviolet absorber (A) is 85 parts or more by mass in total 100 parts by mass of ultraviolet absorbers.
[4] The two-pack type dental composition according to [2] or [3], wherein R¹ in the ultraviolet absorber (A) is an alkyl group or alkoxy group having 1 to 15 carbon atoms.
[5] The two-pack type dental composition according to [4], wherein R¹ in the ultraviolet absorber (A) is a branched alkyl group or alkoxy group having 3 to 15 carbon atoms.
[6] The two-pack type dental composition according to [5], wherein R¹ in the ultraviolet absorber (A) is a 2-ethylhexyloxy group.
[7] The two-pack type dental composition according to any one of [1] to [6], wherein the ultraviolet absorber (A) is bisethylhexyloxyphenol methoxyphenyl triazine and/or 2-[4,6-bis(biphenyl-4-yl)-1,3,5-triazin-2-yl]-5-[(2-ethylhexyl)oxy]phenol.
[8] The two-pack type dental composition according to any one of [1] to [7], wherein the chemical polymerization initiator (C) is a peroxide.
[9] The two-pack type dental composition according to any one of [1] to [8], wherein the chemical polymerization accelerator (D) is at least one selected from the group consisting of an aromatic sulfinic acid and a salt thereof, a sulfur-containing reducing inorganic compound, a thiourea compound, an ascorbic acid compound, a benzotriazole compound, a benzoimidazole compound, and a transition metal compound.
[10] The two-pack type dental composition according to any one of [1] to [9], which further comprises a photopolymerization initiator (E).
[11] The two-pack type dental composition according to any one of [1] to [10], which further comprises a filler (F).

### Advantageous Effects of Invention

A two-pack type dental composition of the present invention undergoes little color change, and exhibits excellent color stability over extended periods.

Given the possibility of dental materials being exposed to high temperatures during transport or an increasing demand for products that do not require refrigerated storage, a two-pack type dental composition of the present invention exhibits color stability even at elevated temperatures.

Moreover, a two-pack type dental composition of the present invention leaves a small amount of material as an unpolymerized layer after curing.

### DESCRIPTION OF EMBODIMENTS

A two-pack type dental composition of the present invention comprises an ultraviolet absorber (A), a polymerizable monomer (B), a chemical polymerization initiator (C), and a chemical polymerization accelerator (D), wherein the ultraviolet absorber (A) is a triazine-based ultraviolet absorber having a maximum absorption wavelength in a wavelength range of 310 nm or more and 400 nm or less.

As used herein, "(meth)acryl" is a collective term for methacryl and acryl.

As used herein, "total 100 parts by mass" in relation to the content of each component refers to the total mass of that component contained in the two agents.

### [Ultraviolet Absorber (A)]

The ultraviolet absorber (A) used in a two-pack type dental composition of the present invention is a triazine-based ultraviolet absorber having a maximum absorption wavelength in a wavelength range of 310 nm or more and 400 nm or less.

The method of measurement of maximum absorption wavelength is as described in the EXAMPLES below.

The following speculation has been made as to why a two-pack type dental composition of the present invention exhibits excellent long-term color stability.

The triazine-based ultraviolet absorber (A) used in the present invention is less likely to form quinoid structures or complexes compared with conventional ultraviolet absorbers because of its structural features, such as phenyl groups providing steric hindrance, even in the presence of coexisting components such as chemical polymerization initiators and chemical polymerization accelerators within the composition. Furthermore, because the triazine-based ultraviolet absorber (A) itself has a maximum absorption wavelength in a wavelength range of 310 nm or more and 400 nm or less, the peak wavelength does not significantly shift toward the longer wavelength side of the photoabsorption spectrum, thereby reducing color change.

Moreover, upon coordination to a catalyst metal as a ligand during prolonged storage, the peak wavelength of the triazine-based ultraviolet absorber (A) does not significantly shift toward the longer wavelength side of the photoabsorption spectrum, leading to reduced color change. This makes it possible to reduce discoloration and adverse effects on curability over the long term compared to when other ultraviolet absorbers are used.

In addition, the triazine-based ultraviolet absorber (A) used in the present invention is highly stable to heat, possibly contributing to excellent color stability over extended periods.

In view of even superior long-term color stability, the ultraviolet absorber (A) is preferably a triazine-based ultraviolet absorber represented by the following general formula (1). 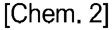 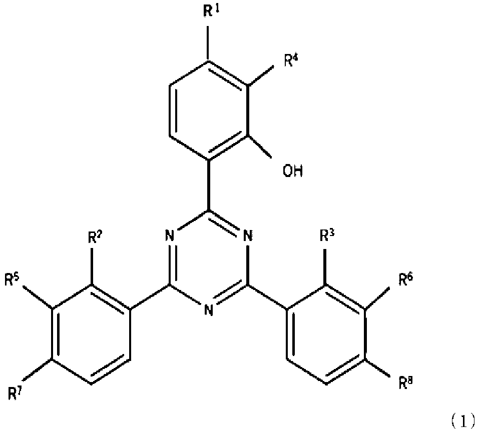 wherein R¹ represents an alkyl group or an alkoxy group, R² and R³ are the same or different from each other and each represent a hydrogen atom, a hydroxyl group, an alkyl group, an alkoxy group, or a halogen atom, R⁴, R⁵, and R⁶ are the same or different from each other and each represent a hydrogen atom or an alkyl group, and R⁷ and R⁸ are the same or different from each other and each represent a hydrogen atom, a hydroxyl group, an alkyl group, an alkoxy group, an aryl group, or a halogen atom, where the alkyl groups and alkoxy groups of R¹ to R⁸ may be optionally substituted.

The alkyl groups represented by R¹ to R⁸ may be linear, branched, or cyclic.

Examples of the alkyl groups include methyl groups, ethyl groups, n-propyl groups, isopropyl groups, cyclopropyl groups, n-butyl groups, isobutyl groups, sec-butyl groups, tert-butyl groups, cyclobutyl groups, n-pentyl groups, isopentyl groups, neopentyl groups, tert-pentyl groups, cyclopentyl groups, n-hexyl groups, isohexyl groups, cyclohexyl groups, n-heptyl groups, cycloheptanyl groups, n-octyl groups, 2-ethylhexyl groups, cyclooctyl groups, n-nonyl groups, cyclononyl groups, and n-decyl groups.

The alkyl group has preferably 1 to 15 carbon atoms, more preferably 1 to 12 carbon atoms, even more preferably 1 to 10 carbon atoms, particularly preferably 1 to 8 carbon atoms.

The branched or cyclic alkyl group has preferably 3 to 15 carbon atoms, more preferably 3 to 12 carbon atoms, even more preferably 3 to 10 carbon atoms, particularly preferably 3 to 8 carbon atoms.

In view of even superior long-term color stability, R¹ is preferably an alkyl group or alkoxy group having 1 to 15 carbon atoms, more preferably a branched alkyl group or alkoxy group having 3 to 15 carbon atoms, even more preferably a 2-ethylhexyloxy group.

The alkoxy groups represented by R¹ to R³ and R⁷ and R⁸ may be linear, branched, or cyclic. Preferably, the alkoxy groups represented by R¹ to R³ and R⁷ and R⁸ are branched.

Examples of the alkoxy groups include methoxy groups, ethoxy groups, n-propoxy groups, isopropoxy groups, n-butoxy groups, tert-butoxy groups, n-hexyloxy groups, cyclohexyloxy groups, n-octyloxy groups, and 2-ethylhexyloxy groups.

The alkoxy group has preferably 1 to 15 carbon atoms, more preferably 1 to 12 carbon atoms, even more preferably 1 to 10 carbon atoms, particularly preferably 1 to 8 carbon atoms.

The branched or cyclic alkoxy group has preferably 3 to 15 carbon atoms, more preferably 3 to 12 carbon atoms, even more preferably 3 to 10 carbon atoms, particularly preferably 3 to 8 carbon atoms.

Examples of the aryl groups represented by R⁷ and R⁸ include phenyl groups, naphthyl groups, and anthryl groups. The aryl group has preferably 6 to 14 carbon atoms, more preferably 6 to 10 carbon atoms.

Examples of the halogen atoms represented by R² and R³ and R⁷ and R⁸ include a fluorine atom, a chlorine atom, a bromine atom, and an iodine atom.

The alkyl groups and alkoxy groups of R¹ to R⁸ may be optionally substituted.

The substituent may be, for example, a halogen atom (a fluorine atom, a chlorine atom, a bromine atom, or an iodine atom), a hydroxyl group, an alkoxy group having 1 to 6 carbon atoms, a dialkylamino group having 1 to 6 carbon atoms on both alkyl groups, or an amino group.

The alkyl groups and alkoxy groups of R¹ to R⁸ may be unsubstituted.

In view of curability, initial color stability, and long-term color stability, the ultraviolet absorber (A) is preferably a triazine-based ultraviolet absorber represented by the general formula (1) above, and that has a maximum absorption wavelength in a wavelength range of 310 nm or more and 400 nm or less.

In view of more easily reducing color change by preventing the peak wavelength from significantly shifting toward the longer wavelength side of the photoabsorption spectrum upon coordination to a catalyst metal as a ligand during prolonged storage, the ultraviolet absorber (A) exhibits a maximum absorption wavelength in a wavelength range of preferably 390 nm or less, more preferably 385 nm or less, even more preferably 380 nm or less, particularly preferably 375 nm or less.

Specific examples of the ultraviolet absorber (A) include 2,4,6-tris[4-(hexyloxy)-2-hydroxy-3-methylphenyl]-1,3,5-triazine, 2,4-bis(2,4-dimethylphenyl)-6-(2-hydroxy-4-n-octyloxyphenyl)-1,3,5-triazine, 2,4,6-tris(2,4-dihydroxyphenyl)-1,3,5-triazine, bisethylhexyloxyphenol methoxyphenyl triazine, and 2-[4,6-bis(biphenyl-4-yl)-1,3,5-triazin-2-yl]-5-[(2-ethylhexyl)oxy]phenol. Preferred are bisethylhexyloxyphenol methoxyphenyl triazine and/or 2-[4,6-bis(biphenyl-4-yl)-1,3,5-triazin-2-yl]-5-[(2-ethylhexyl)oxy]phenol.

The ultraviolet absorber (A) may be used alone, or two or more thereof may be used in combination.

In view of enhancing the long-term color stability due to the structural features of the triazine-based ultraviolet absorber (A) (e.g., steric hindrance attributable to phenyl groups) and the maximum absorption wavelength occurring in a wavelength range of 310 nm or more and 400 nm or less in the triazine-based ultraviolet absorber (A) itself, the content of ultraviolet absorber (A) is preferably 85 parts or more by mass, more preferably 90 parts or more by mass, even more preferably 95 parts or more by mass, yet more preferably 98 parts or more by mass, particularly preferably 100 parts by mass in total 100 parts by mass of the ultraviolet absorbers contained in a two-pack type dental composition of the present invention.

In calculating a total of 100 parts by mass of the ultraviolet absorbers contained in the two-pack type dental composition, benzotriazole compounds represented by the general formula (2) below and benzoimidazole compounds represented by the general formula (3) below are not counted as ultraviolet absorbers.

In view of curability, initial color stability, and long-term color stability, the content of ultraviolet absorber (A) is preferably 0.0001 mass% or more, more preferably 0.001 mass% or more, even more preferably 0.005 mass% or more, particularly preferably 0.01 mass% or more in 100 mass% of the total mass of a two-pack type dental composition of the present invention.

In view of curability, initial color stability, and long-term color stability, the content of ultraviolet absorber (A) is preferably 1 mass% or less, more preferably 0.8 mass% or less, even more preferably 0.5 mass% or less, particularly preferably 0.2 mass% or less in 100 mass% of the total mass of a two-pack type dental composition of the present invention.

In view of curability, initial color stability, and long-term color stability, the content of ultraviolet absorber (A) is preferably 0.001 parts or more by mass, more preferably 0.01 parts or more by mass, even more preferably 0.05 parts or more by mass, particularly preferably 0.1 parts or more by mass relative to total 100 parts by mass of polymerizable monomer (B).

In view of curability, initial color stability, and long-term color stability, the content of ultraviolet absorber (A) is preferably 5 parts or less by mass, more preferably 1 part or less by mass, even more preferably 0.8 parts or less by mass, particularly preferably 0.5 parts or less by mass relative to total 100 parts by mass of polymerizable monomer (B).

### [Polymerizable Monomer (B)]

Preferred for use as the polymerizable monomer (B) used in a two-pack type dental composition of the present invention are radical polymerizable monomers.

Examples of radical polymerizable monomers as polymerizable monomer (B) include (meth)acrylate polymerizable monomers, (meth)acrylamide polymerizable monomers, and esters, vinyl esters, and vinyl ethers of acids such as α-cyanoacrylic acid, (meth)acrylic acid, α-halogenated acrylic acid, crotonic acid, cinnamic acid, sorbic acid, maleic acid, and itaconic acid, as well as mono-N-vinyl derivatives and styrene derivatives. In view of curability, preferred among these are (meth)acrylate polymerizable monomers and (meth)acrylamide polymerizable monomers.

The polymerizable monomer (B) can be broadly classified into polymerizable monomer having an acidic group (B-1), and polymerizable monomer having no acidic group (B-2).

The polymerizable monomer (B) may be used alone, or two or more thereof may be used in combination.

### <Polymerizable Monomer Having Acidic Group (B-1)>

A two-pack type dental composition of the present invention may comprise a polymerizable monomer having an acidic group (B-1).

The polymerizable monomer having an acidic group (B-1) used in the present invention is preferably a radical polymerizable monomer having at least one acidic group and polymerizable group. In view of adhesive properties, the acidic group is preferably, for example, a phosphoric acid group, a pyrophosphoric acid group, a thiophosphoric acid group, a phosphonic acid group, a carboxylic acid group, or a sulfonic acid group.

In view of ease of radical polymerization, preferred as polymerizable groups are (meth)acryloyloxy groups and/or (meth)acrylamide groups.

The polymerizable monomer having an acidic group (B-1) may be incorporated alone, or two or more thereof may be used in combination.

Examples of polymerizable monomers having a phosphoric acid group include 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, bis[2-(meth)acryloyloxyethyl] hydrogen phosphate, bis[4-(meth)acryloyloxybutyl] hydrogen phosphate, bis[6-(meth)acryloyloxyhexyl] hydrogen phosphate, bis[8-(meth)acryloyloxyoctyl] hydrogen phosphate, bis[9-(meth)acryloyloxynonyl] hydrogen phosphate, bis[10-(meth)acryloyloxydecyl] hydrogen phosphate, 1,3-di(meth)acryloyloxypropyl dihydrogen phosphate, 2-(meth)acryloyloxyethylphenyl hydrogen phosphate, 2-(meth)acryloyloxyethyl-(2-bromoethyl) hydrogen phosphate, 2-(meth)acryloyloxyethyl-(4-methoxyphenyl) hydrogen phosphate, and 2-(meth)acryloyloxypropyl-(4-methoxyphenyl) hydrogen phosphate, and acid chlorides, alkali metal salts, and amine salts of these.

Examples of polymerizable monomers having a pyrophosphoric acid group include bis[2-(meth)acryloyloxyethyl] pyrophosphate, bis[4-(meth)acryloyloxybutyl] pyrophosphate, bis[6-(meth)acryloyloxyhexyl] pyrophosphate, bis[8-(meth)acryloyloxyoctyl] pyrophosphate, and bis[10-(meth)acryloyloxydecyl] pyrophosphate, and acid chlorides, alkali metal salts, and amine salts of these.

Examples of polymerizable monomers having a thiophosphoric acid group include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, and 20-(meth)acryloyloxyicosyl dihydrogen thiophosphate, and acid chlorides, alkali metal salts, and ammonium salts of these.

Examples of polymerizable monomers having a phosphonic acid group include 2-(meth)acryloyloxyethylphenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexylphosphonoacetate, and 10-(meth)acryloyloxydecyl-3-phosphonoacetate, and acid chlorides, alkali metal salts, and ammonium salts of these.

Examples of polymerizable monomers having a carboxylic acid group include monofunctional (meth)acrylic acid esters having one carboxyl group or an acid anhydride group thereof within the molecule, monofunctional (meth)acrylic acid esters having multiple carboxyl groups or acid anhydride groups thereof within the molecule, and acid chlorides, alkali metal salts, and ammonium salts of these.

Examples of the monofunctional polymerizable monomers having one carboxyl group or an acid anhydride group thereof within the molecule include (meth)acrylic acid, N-(meth)acryloyl glycine, N-(meth)acryloyl aspartic acid, 2-(meth)acryloyloxyethyl hydrogen succinate, 2-(meth)acryloyloxyethyl hydrogen phthalate, 2-(meth)acryloyloxyethyl hydrogen malate, O-(meth)acryloyl tyrosine, N-(meth)acryloyl tyrosine, N-(meth)acryloyl phenylalanine, N-(meth)acryloyl-p-aminobenzoic acid, N-(meth)acryloyl-o-aminobenzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, N-(meth)acryloyl-4-aminosalicylic acid, and compounds derived by converting the carboxyl group of the aforementioned compounds into an acid anhydride group.

Examples of the monofunctional polymerizable monomers having multiple carboxyl groups or acid anhydride groups thereof within the molecule include 6-(meth)acryloyloxyhexane-1,1-dicarboxylic acid, 9-(meth)acryloyloxynonane-1,1-dicarboxylic acid, 10-(meth)acryloyloxydecane-1,1-dicarboxylic acid, 11-(meth)acryloyloxyundecane-1,1-dicarboxylic acid, 12-(meth)acryloyloxydodecane-1,1-dicarboxylic acid, 13-(meth)acryloyloxytridecane-1,1-dicarboxylic acid, 4-(meth)acryloyloxyethyl trimellitate, 4-(meth)acryloyloxyethyl trimellitate anhydride, 4-(meth)acryloyloxybutyl trimellitate, 4-(meth)acryloyloxyhexyl trimellitate, 4-(meth)acryloyloxydecyl trimellitate, 2-(meth)acryloyloxyethyl-3'-(meth)acryloyloxy-2'-(3,4-dicarboxybenzoyloxy)propyl succinate, 6-(meth)acryloyloxyethylnaphthalene-1,2,6-tricarboxylic anhydride, 6-(meth)acryloyloxyethylnaphthalene-2,3,6-tricarboxylic anhydride, 4-(meth)acryloyloxyethylcarbonylpropionoyl-1,8-naphthalic anhydride, and 4-(meth)acryloyloxyethylnaphthalene-1,8-tricarboxylic anhydride.

Examples of polymerizable monomers having a sulfonic acid group include 2-sulfoethyl (meth)acrylate, and acid chlorides, alkali metal salts, and ammonium salts thereof.

In view of providing favorable bond strength as a two-pack type dental composition, preferred among these polymerizable monomers having an acidic group (B-1) are polymerizable monomers having a phosphoric acid group, or polymerizable monomers having a carboxylic acid group, more preferably 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, 4-(meth)acryloyloxyethyl trimellitate anhydride, 4-(meth)acryloyloxyethyl trimellitate, 11-(meth)acryloyloxyundecane-1,1-dicarboxylic acid, and a mixture of 2-(meth)acryloyloxyethyl dihydrogen phosphate and bis(2-methacryloyloxyethyl) hydrogen phosphate, even more preferably 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, and 20-(meth)acryloyloxyicosyl dihydrogen phosphate. In view of a balance with curability, 10-(meth)acryloyloxydecyl dihydrogen phosphate is most preferred.

In view of adhesive properties to tooth structure, the content of the polymerizable monomer having an acidic group (B-1) in a two-pack type dental composition of the present invention is preferably 0 to 30 parts by mass, more preferably 0 to 20 parts by mass, even more preferably 0 to 15 parts by mass in total 100 parts by mass of polymerizable monomer (B).

### <Polymerizable Monomer Having no Acidic Group (B-2)>

In the present invention, the polymerizable monomer having no acidic group (B-2) can be classified into hydrophilic polymerizable monomer having no acidic group and that has a solubility of 4.5 g/L or more in 25°C water (B-2a), and hydrophobic polymerizable monomer having no acidic group and that has a solubility of less than 4.5 g/L in 25°C water (B-2b).

The polymerizable monomer having no acidic group (B-2) may be incorporated alone, or two or more thereof may be used in combination.

### · Hydrophilic Polymerizable Monomer Having no Acidic Group (B-2a)

In a two-pack type dental composition of the present invention, the hydrophilic polymerizable monomer having no acidic group (B-2a) (hereinafter, also referred to simply as "hydrophilic polymerizable monomer (B-2a)") in the polymerizable monomer (B) is preferably less than 15 parts by mass in total 100 parts by mass of the polymerizable monomer (B).

The hydrophilic polymerizable monomer (B-2a) refers to monomers that do not have acidic groups and have a solubility of 4.5 g/L or more in 25°C water.

In a two-pack type dental composition of the present invention, in view of satisfying both paste flowability and formability, the monomer content specified as a solubility in water is preferably less than 15 parts by mass in total 100 parts by mass of the polymerizable monomer (B).

The hydrophilic polymerizable monomer (B-2a) may be used alone, or two or more thereof may be used in combination.

When the content of the hydrophilic polymerizable monomer (B-2a) in a two-pack type dental composition of the present invention is 15 parts or more by mass in total 100 parts by mass of the polymerizable monomer (B), the paste flowability decreases, making it impossible to achieve both flowability and formability. Accordingly, the content of the hydrophilic polymerizable monomer (B-2a) in a two-pack type dental composition of the present invention is preferably 10 parts or less by mass, more preferably 5 parts or less by mass in total 100 parts by mass of the polymerizable monomer (B). The content of hydrophilic polymerizable monomer (B-2a) may be 0 parts by mass in total 100 parts by mass of the polymerizable monomer (B).

The hydrophilic polymerizable monomer (B-2a) improves the wettability of the two-pack type dental composition to tooth structure. Preferred as hydrophilic polymerizable monomer (B-2a) are radical polymerizable monomers lacking acidic groups and having polymerizable groups. In view of ease of radical polymerization, the polymerizable groups are preferably (meth)acryloyloxy groups and/or (meth)acrylamide groups. Examples of the hydrophilic polymerizable monomer (B-2a) include:
hydrophilic (meth)acrylate monofunctional polymerizable monomers such as 2-hydroxyethyl (meth)acrylate, 3-hydroxypropyl (meth)acrylate, 2-hydroxypropyl (meth)acrylate, 1,3-dihydroxypropyl (meth)acrylate, 2,3-dihydroxypropyl (meth)acrylate, 2-((meth)acryloyloxy)ethyltrimethylammonium chloride, and tetrahydrofurfuryl (meth)acrylate;
hydrophilic (meth)acrylate polyfunctional polymerizable monomers such as triethylene glycol di(meth)acrylate, tetraethylene glycol di(meth)acrylate, nonaethylene glycol di(meth)acrylate, 1,3-bis(methacryloyloxy)-2-propanol, and 1,2-bis(3-methacryloyloxy-2-hydroxypropoxy)ethane; and
hydrophilic (meth)acrylamide polymerizable monomers such as N-methylol(meth)acrylamide, N-hydroxyethyl(meth)acrylamide, N,N-bis(2-hydroxyethyl)(meth)acrylamide, N-methoxymethyl(meth)acrylamide, N-ethoxymethyl(meth)acrylamide, diacetone(meth)acrylamide, 4-(meth)acryloylmorpholine, N-trihydroxymethyl-N-methyl(meth)acrylamide, N,N-dimethylacrylamide, N,N-diethylacrylamide, and N-methacryloyloxyethylacrylamide.

In view of curability, preferred for use among these hydrophilic polymerizable monomers (B-2a) are (meth)acrylate polyfunctional polymerizable monomers, more preferably triethylene glycol dimethacrylate (commonly known as 3G), 1,3-bis(methacryloyloxy)-2-propanol, and 1,2-bis(3-methacryloyloxy-2-hydroxypropoxy)ethane (commonly known as #801), even more preferably 3G and #801.

### · Hydrophobic Polymerizable Monomer Having no Acidic Group (B-2b)

In a two-pack type dental composition of the present invention, the polymerizable monomer (B) comprises a hydrophobic polymerizable monomer having no acidic group (B-2b) (hereinafter, also referred to simply as "hydrophobic polymerizable monomer (B-2b)"). Here, the hydrophobic polymerizable monomer (B-2b) refers to monomers that do not have acidic groups and have a solubility of less than 4.5 g/L in 25°C water. The use of hydrophobic polymerizable monomer (B-2b) weakens hydrophilic interactions between the polymerizable monomer and the filler (F) when combined with the filler (F), allowing the paste flowability to improve. By using hydrophobic polymerizable monomer (B-2b), it is also possible to reduce the stringiness (stringing tendency) of the two-pack type dental composition and improve properties such as the mechanical strength of the cured product.

The hydrophobic polymerizable monomer (B-2b) may be incorporated alone, or two or more thereof may be used in combination.

Preferred as hydrophobic polymerizable monomer (B-2b) are radical polymerizable monomers lacking acidic groups and having polymerizable groups. In view of ease of radical polymerization, the polymerizable groups are preferably (meth)acryloyloxy groups and/or (meth)acrylamide groups. Examples of the hydrophobic polymerizable monomer (B-2b) include hydrophobic monofunctional polymerizable monomers, and crosslinkable polymerizable monomers such as hydrophobic aromatic bifunctional polymerizable monomers, hydrophobic aliphatic bifunctional polymerizable monomers, and hydrophobic tri- and higher-functional polymerizable monomers.

Examples of the hydrophobic monofunctional polymerizable monomers include methyl (meth)acrylate, ethyl (meth)acrylate, butyl (meth)acrylate, benzyl (meth)acrylate, isobornyl (meth)acrylate, p-cumyl-phenoxyethylene glycol (meth)acrylate, m-phenoxybenzyl (meth)acrylate, stearyl (meth)acrylate, dicyclopentanyl (meth)acrylate, butoxydiethylene glycol (meth)acrylate, and methoxypolyethylene glycol (meth)acrylate. Preferred among these are benzyl methacrylate, isobornyl methacrylate, and p-cumyl-phenoxyethylene glycol methacrylate.

Examples of the hydrophobic aromatic bifunctional polymerizable monomers include 2,2-bis((meth)acryloyloxyphenyl)propane, 2,2-bis[4-(2-hydroxy-3-(meth)acryloyloxypropoxy)phenyl]propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, and 2,2-bis(4-(meth)acryloyloxyisopropoxyphenyl)propane. Preferred among these are 2,2-bis[4-(2-hydroxy-3-methacryloyloxypropoxy)phenyl]propane (commonly known as Bis-GMA), 2,2-bis(4-methacryloyloxyethoxyphenyl)propane, 2,2-bis(4-methacryloyloxypolyethoxyphenyl)propane (with an average of 2.6 moles of ethoxy group added; commonly known as D-2.6E), 2,2-bis(4-methacryloyloxydiethoxyphenyl)propane, 2,2-bis(4-methacryloyloxytriethoxyphenyl)propane, 2,2-bis(4-methacryloyloxytetraethoxyphenyl)propane, and 2,2-bis(4-methacryloyloxypentaethoxyphenyl)propane. More preferred are Bis-GMA and D-2.6E.

Examples of the aliphatic bifunctional polymerizable monomers include monoethylene glycol di(meth)acrylate, diethylene glycol di(meth)acrylate, propylene glycol di(meth)acrylate, butylene glycol di(meth)acrylate, neopentyl glycol di(meth)acrylate, 1,5-pentanediol di(meth)acrylate, 1,6-hexanediol di(meth)acrylate, 1,10-decanediol di(meth)acrylate, and 2,2,4-trimethylhexamethylenebis(2-carbamoyloxyethyl) di(meth)acrylate. Preferred among these are neopentyl glycol dimethacrylate (commonly known as NPG), 2,2,4-trimethylhexamethylenebis(2-carbamoyloxyethyl) dimethacrylate (commonly known as UDMA), 1,10-decanediol dimethacrylate (commonly known as DD), and 2,2,4-trimethylhexamethylenebis(2-carbamoyloxyethyl) dimethacrylate. More preferred are NPG, UDMA, and DD.

Examples of the tri- and higher-functional polymerizable monomers include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis[2-(aminocarboxy)propane-1,3-diol]tetra(meth)acrylate, and 1,7-diacryloyloxy-2,2,6,6-tetra(meth)acryloyloxymethyl-4-oxaheptane. Preferred among these is N,N-(2,2,4-trimethylhexamethylene)bis[2-(aminocarboxy)propane-1,3-diol]tetramethacrylate.

In view of handling properties and the mechanical strength of the cured product, preferred among these hydrophobic polymerizable monomers (B-2b) are aromatic bifunctional polymerizable monomers, and aliphatic bifunctional polymerizable monomers.

When the content of the hydrophobic polymerizable monomer (B-2b) in a two-pack type dental composition of the present invention is excessively high, it may lead to a decrease in the wettability of the two-pack type dental composition to tooth structure, resulting in reduced adhesion. In contrast, an excessively low content of hydrophobic polymerizable monomer (B-2b) may result in increased hydrophilic interactions between the polymerizable monomer and the filler, leading to insufficient paste flowability and insufficient mechanical strength in the cured product.

Accordingly, the content of the hydrophobic polymerizable monomer (B-2b) in a two-pack type dental composition of the present invention is preferably 55 parts or more by mass, more preferably 65 parts or more by mass, even more preferably 70 parts or more by mass, yet more preferably 85 parts or more by mass, particularly preferably 90 parts or more by mass in total 100 parts by mass of the polymerizable monomer (B). The content of hydrophobic polymerizable monomer (B-2b) may be 100 parts by mass.

### [Chemical Polymerization Initiator (C)]

A two-pack type dental composition of the present invention comprises a chemical polymerization initiator (C).

Examples of chemical polymerization initiator (C) include peroxides (organic peroxides, inorganic peroxides). These are not particularly limited, and known peroxides may be used.

The chemical polymerization initiator (C) may be used alone, or two or more thereof may be used in combination.

Typical examples of organic peroxides include hydroperoxides, peroxyesters, ketone peroxides, peroxyketals, dialkyl peroxides, diacyl peroxides, and peroxydicarbonates. Particularly preferred among these are hydroperoxides and peroxyesters. In view of reducing variation in working time even after prolonged storage of a two-pack type dental composition of the present invention, peroxyesters are most preferred.

Specific examples of hydroperoxides include cumene hydroperoxide, t-butyl hydroperoxide, t-hexyl hydroperoxide, p-menthane hydroperoxide, diisopropylbenzene hydroperoxide, and 1,1,3,3-tetramethylbutyl hydroperoxide (hereinafter, also referred to by the abbreviation "THP").

The peroxyesters may be any known peroxyesters with no limitation, provided that the peroxy group (-OO- group) has an acyl group at one end, and a hydrocarbon group (or a similar group) at the other end. Specific examples include α,α-bis(neodecanoylperoxy)diisopropylbenzene, cumyl peroxyneodecanoate, 1,1,3,3-tetramethylbutyl peroxyneodecanoate, 1-cyclohexyl-1-methylethyl peroxyneodecanoate, t-hexyl peroxyneodecanoate, t-butyl peroxyneodecanoate, t-hexyl peroxypivalate, t-butyl peroxypivalate, 1,1,3,3-tetramethylbutyl peroxy-2-ethylhexanoate, 2,5-dimethyl-2,5-bis(2-ethylhexanoylperoxy)hexane, 1-cyclohexyl-1-methylethyl peroxy-2-ethylhexanoate, t-hexyl peroxy-2-ethylhexanoate, t-butyl peroxy-2-ethylhexanoate, t-butyl peroxyisobutyrate, t-hexyl peroxyisopropyl monocarbonate, t-butyl peroxymaleic acid, t-butyl peroxy-3,5,5-trimethylhexanoate, t-butyl peroxylaurate, 2,5-dimethyl-2,5-bis(m-toluoylperoxy)hexane, t-butyl peroxyisopropyl monocarbonate, t-butyl peroxy-2-ethylhexyl monocarbonate, t-hexyl peroxybenzoate, 2,5-dimethyl-2,5-bis(benzoylperoxy)hexane, t-butyl peroxyacetate, t-butyl peroxy-m-toluoylbenzoate, t-butyl peroxybenzoate (hereinafter, also referred to by the abbreviation "BPB"), and bis(t-butylperoxy)isophthalate. In view of storage stability and reactivity, preferred among these are t-butyl peroxymaleic acid, t-butyl peroxy-3,5,5-trimethylhexanoate, t-butyl peroxybenzoate, t-butyl peroxyisopropyl monocarbonate, t-butyl peroxy-2-ethylhexyl monocarbonate, and t-butyl peroxyacetate, more preferably t-butyl peroxybenzoate.

Examples of ketone peroxides include methyl ethyl ketone peroxide, cyclohexanone peroxide, methyl cyclohexanone peroxide, methyl acetoacetate peroxide, and acetyl acetone peroxide.

Examples of peroxyketals include 1,1-bis(t-hexylperoxy)3,3,5-trimethylcyclohexane, 1,1-bis(t-hexylperoxy)cyclohexane, 1,1-bis(t-butylperoxy)3,3,5-trimethylcyclohexanone, 1,1-bis(t-butylperoxy)cyclohexane, 1,1-bis(t-butylperoxy)cyclodecane, 2,2-bis(t-butylperoxy)butane, n-butyl 4,4-bis(t-butylperoxy)valerate, and 2,2-bis(4,4-di-t-butylperoxycyclohexyl)propane.

Examples of dialkyl peroxides include α,α-bis(t-butylperoxy)diisopropylbenzene, dicumyl peroxide, 2,5-dimethyl-2,5-bis(t-butylperoxy)hexane, t-butyl cumyl peroxide, di-t-butyl peroxide, and 2,5-dimethyl-2,5-bis(t-butylperoxy)3-hexyne.

Examples of diacyl peroxides include isobutyryl peroxide, 2,4-dichlorobenzoyl peroxide, 3,5,5-trimethylhexanoyl peroxide, octanoyl peroxide, lauroyl peroxide, stearyl peroxide, succinic acid peroxide, m-toluoylbenzoyl peroxide, and benzoyl peroxide.

Examples of peroxydicarbonates include di-n-propylperoxy dicarbonate, diisopropylperoxy dicarbonate, bis(4-t-butylcyclohexyl)peroxydicarbonate, di(2-ethoxyethyl)peroxydicarbonate, di(2-ethylhexyl)peroxydicarbonate, di(2-methoxybutyl)peroxydicarbonate, and di(3-methyl-3-methoxybutyl)peroxydicarbonate.

Examples of inorganic peroxides include peroxydisulfates and peroxydiphosphates. In view of curability, peroxydisulfates are preferred. Specific examples of peroxydisulfates include sodium peroxydisulfate, potassium peroxydisulfate (hereinafter, also referred to by the abbreviation "KPS"), aluminum peroxydisulfate, and ammonium peroxydisulfate.

In view of curability, the content of organic peroxides and inorganic peroxides is preferably 0.01 to 5 parts by mass, more preferably 0.05 to 2 parts by mass in total 100 parts by mass of the polymerizable monomer (B) in a two-pack type dental composition of the present invention.

### [Chemical Polymerization Accelerator (D)]

Examples of the chemical polymerization accelerator (D) used in a two-pack type dental composition of the present invention include aliphatic amines, aromatic amines having no electron withdrawing group on the aromatic ring, transition metal compounds (period 4 transition metal compounds, and transition metal compounds outside of period 4), thiourea compounds, tin compounds, aromatic sulfinic acids and salts thereof, benzotriazole compounds, benzoimidazole compounds, bromides, borate compounds, barbituric acid compounds, ascorbic acid compounds, and sulfur-containing reducing inorganic compounds. Preferably, the chemical polymerization accelerator (D) is at least one selected from the group consisting of an aromatic sulfinic acid and a salt thereof, a sulfur-containing reducing inorganic compound, a thiourea compound, an ascorbic acid compound, a benzotriazole compound, a benzoimidazole compound, and a transition metal compound.

The chemical polymerization accelerator (D) may be used alone, or two or more thereof may be used in combination.

In a certain embodiment, when the polymerizable monomer (B) does not comprise a polymerizable monomer having an acidic group (B-1), the chemical polymerization accelerator (D) is preferably at least one selected from the group consisting of an aromatic amine having no electron withdrawing group on the aromatic ring, a period 4 transition metal compound, a transition metal compound outside of period 4, and a thiourea compound.

In view of curability and storage stability, the content of chemical polymerization accelerator (D) in such an embodiment is preferably 0.001 to 20 parts by mass, more preferably 0.005 to 10 parts by mass, even more preferably 0.025 to 5 parts by mass relative to total 100 parts by mass of polymerizable monomer (B).

In another certain embodiment, when the polymerizable monomer (B) comprises a polymerizable monomer having an acidic group (B-1), the chemical polymerization accelerator (D) is preferably at least one selected from the group consisting of a copper compound, an aromatic sulfinic acid and a salt thereof, a benzotriazole compound, a benzoimidazole compound, a bromide, an ascorbic acid compound, and a sulfur-containing reducing inorganic compound.

In view of curability and storage stability, the content of chemical polymerization accelerator (D) in such an embodiment is preferably 0.001 to 40 parts by mass, more preferably 0.005 to 20 parts by mass, even more preferably 0.025 to 10 parts by mass relative to total 100 parts by mass of polymerizable monomer (B).

Examples of aliphatic amines include primary aliphatic amines such as n-butylamine, n-hexylamine, and n-octylamine; secondary aliphatic amines such as diisopropylamine, dibutylamine, and N-methylethanolamine; and tertiary aliphatic amines such as N-methyldiethanolamine, N-ethyldiethanolamine, N-n-butyldiethanolamine, N-lauryldiethanolamine, 2-(dimethylamino)ethyl (meth)acrylate, N-methyldiethanolamine di(meth)acrylate, N-ethyldiethanolamine di(meth)acrylate, triethanolamine tri(meth)acrylate, triethanolamine, trimethylamine, triethylamine, and tributylamine. In view of redox reactivity, preferred among these are tertiary aliphatic amines, particularly preferably N-methyldiethanolamine, triethanolamine, and 2-(dimethylamino)ethyl methacrylate.

Examples of aromatic amines having no electron withdrawing group on the aromatic ring include aromatic tertiary amines such as N,N-bis(2-hydroxyethyl)-3,5-dimethylaniline, N,N-bis(2-hydroxyethyl)-p-toluidine (hereinafter, also referred to by the abbreviation "DEPT"), N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, and N,N-dimethyl-3,5-di-t-butylaniline.

The aromatic amines having no electron withdrawing group on the aromatic ring are used as reducing agents for redox polymerization initiators.

In view of redox reactivity, preferred among these is N,N-bis(2-hydroxyethyl)-p-toluidine.

The period 4 transition metal compound may be any of a vanadium compound, a copper compound, or a period 4 transition metal compound excluding vanadium and copper (hereinafter, also referred to simply as "other period 4 transition metal compounds").

In a certain embodiment, in view of accelerating polymerization, the chemical polymerization accelerator (D) preferably comprises a vanadium compound and a copper compound.

Examples of vanadium compounds include vanadyl(IV) acetylacetonate, vanadyl stearate, vanadium naphthenate, vanadium benzoylacetonate, vanadyl oxalate, bis(maltolato)oxovanadium(IV), oxobis(1-phenyl-1,3-butanedionate)vanadium(IV), vanadium(V) oxytriisopropoxide, ammonium metavanadate, sodium metavanadate, vanadium(V) oxide, vanadium(IV) oxide, and vanadyl(IV) sulfate. In view of properties such as the solubility in solvent, preferred are vanadyl(IV) acetylacetonate, and bis(maltolato)oxovanadium(IV), more preferably vanadyl(IV) acetylacetonate.

In view of curability, the content of vanadium compounds is preferably 0.005 to 1 parts by mass relative to total 100 parts by mass of the polymerizable monomer (B) in a two-pack type dental composition of the present invention.

Preferably, the copper compounds are, for example, compounds that are soluble in radical polymerizable monomers.

Examples of the copper compounds include copper carboxylates, copper β-diketones, copper β-ketoesters, copper alkoxides, copper dithiocarbamates, and salts of copper and inorganic acids.

Examples of copper carboxylates include copper acetate, copper isobutyrate, copper gluconate, copper citrate, copper phthalate, copper tartarate, copper oleate, copper octylate, copper octenate, copper naphthenate, copper methacrylate, and copper 4-cyclohexylbutyrate.

Examples of copper β-diketones include copper acetylacetonate, copper trifluoroacetylacetonate, copper hexafluoroacetylacetonate, copper 2,2,6,6-tetramethyl-3,5-heptanedionate, and copper benzoylacetonate.

Examples of copper β-ketoesters include copper ethyl acetoacetate.

Examples of copper alkoxides include copper methoxide, copper ethoxide, copper isopropoxide, copper 2-(2-butoxyethoxy)ethoxide, and copper 2-(2-methoxyethoxy)ethoxide.

Examples of copper dithiocarbamates include copper dimethyl dithiocarbamate.

Examples of salts of copper and inorganic acids include copper nitrate, and copper chloride.

In view of solubility and reactivity to polymerizable monomer (B), preferred are copper carboxylates, copper β-diketones, and copper β-ketoesters, more preferably copper acetate, and copper acetylacetonate.

In view of curability, the content of copper compounds is preferably 0.000005 to 1 parts by mass, more preferably 0.00001 to 0.5 parts by mass, even more preferably 0.0001 to 0.1 parts by mass relative to total 100 parts by mass of the polymerizable monomer (B) in a two-pack type dental composition of the present invention.

Examples of other period 4 transition metal compounds include scandium isopropoxide, iron(III) ethoxide, titanium ethoxide, titanium ethoxide, titanium isopropoxide, titanium butoxide, titanium hydroxide, and titanium fluoride.

In view of enhanced polymerization accelerating effect, preferred among the period 4 transition metal compounds are vanadyl(IV) acetylacetonate, and bis(maltolato)oxovanadium(IV), more preferably vanadyl(IV) acetylacetonate.

Examples of transition metal compounds outside of period 4 include strontium carbonate, strontium hydroxide, strontium ethoxide, tin(II) methoxide, indium ethoxide, actinium ethoxide, yttrium isopropoxide, lanthanum methoxide, lanthanum ethoxide, lanthanum isopropoxide, lanthanum butoxide, lanthanum hydroxide, lanthanum carbonate, lanthanum fluoride, cerium isopropoxide, praseodymium isopropoxide, promethium isopropoxide, neodymium isopropoxide, samarium isopropoxide, europium isopropoxide, gadolinium isopropoxide, terbium ethoxide, terbium methoxide, dysprosium isopropoxide, holmium isopropoxide, erbium isopropoxide, thulium isopropoxide, ytterbium isopropoxide, zirconium ethoxide, zirconium isopropoxide, zirconium butoxide, tungsten(IV) methoxide, tungsten(IV) isopropoxide, and tungsten(IV) butoxide.

Examples of thiourea compounds include thiourea, methylthiourea, ethylthiourea, ethylenethiourea, N,N'-dimethylthiourea, N,N'-diethylthiourea, N,N'-di-n-propylthiourea, N,N'-dicyclohexylthiourea, trimethylthiourea, triethylthiourea, tri-n-propylthiourea, tricyclohexylthiourea, tetramethylthiourea, tetraethylthiourea, tetra-n-propylthiourea, tetracyclohexylthiourea, 1-(2-pyridyl)-2-thiourea, (6-methyl-pyridin-2-yl)thiourea, and 4,4-dimethylethylenethiourea.

In view of properties such as the solubility in solvent and storage stability, preferred are 1-(2-pyridyl)-2-thiourea, (6-methyl-pyridin-2-yl)thiourea, and 4,4-dimethylethylenethiourea.

Examples of tin compounds include di-n-butyltin dimaleate, di-n-octyltin dimaleate, di-n-octyltin dilaurate, and di-n-butyltin dilaurate. Particularly preferred tin compounds are di-n-octyltin dilaurate and di-n-butyltin dilaurate.

Examples of aromatic sulfinic acids and salts thereof include benzenesulfinic acid, p-toluenesulfinic acid, o-toluenesulfinic acid, ethylbenzenesulfinic acid, decylbenzenesulfinic acid, dodecylbenzenesulfinic acid, 2,4,6-trimethylbenzenesulfinic acid, 2,4,6-triisopropylbenzenesulfinic acid, chlorobenzenesulfinic acid, naphthalenesulfinic acid, and lithium salts, sodium salts, potassium salts, rubidium salts, cesium salts, magnesium salts, calcium salts, strontium salts, iron salts, zinc salts, ammonium salts, tetramethylammonium salts, and tetraethylammonium salts of these. In view of polymerizability and storage stability of the composition, preferred are 2,4,6-trimethylbenzenesulfinic acid, and lithium salts, sodium salts, potassium salts, magnesium salts, and calcium salts of 2,4,6-triisopropylbenzenesulfinic acid, more preferably lithium salts, sodium salts, potassium salts, magnesium salts, and calcium salts of 2,4,6-triisopropylbenzenesulfinic acid.

Preferably, the aromatic sulfinic acids and salts thereof are at least partially dispersed in powder form within the composition. With the aromatic sulfinic acids and salts thereof being dispersed in powder form, it is easier to provide a longer working time margin for a two-pack type dental composition of the present invention, and, when the composition is applied to moist materials such as the tooth structure, the aromatic sulfinic acids and salts thereof dissolve in the water on the surface of the moist material, making it possible to more greatly enhance polymerization both at the bonding interface and within the resin-impregnated layer. When the aromatic sulfinic acids and salts thereof are dispersed as powder, it is preferable that the aromatic sulfinic acids and salts thereof have a solubility in water of 1 mg/100 mL or more at ordinary temperature (25°C). A solubility of 1 mg/100 mL or more facilitates sufficient dissolution of aromatic sulfinic acids and salts thereof in the water on moist materials at the bonding interface in applications where a two-pack type dental composition of the present invention is applied to moist materials, facilitating the effect of dispersing as powder. In view of resistance to settling, the aromatic sulfinic acids and salts thereof have an average particle diameter of preferably 500 µm or less, more preferably 100 µm or less, even more preferably 50 µm or less. In view of preventing excessive increase in the specific surface area of the powder and facilitating in maintaining good ease of handling for the two-pack type dental composition, the average particle diameter is preferably 0.01 µm or more. That is, when dispersed as powder, the aromatic sulfinic acids and salts thereof have an average particle diameter ranging from preferably 0.01 to 500 µm, more preferably from 0.01 to 100 µm. The average particle diameter can be measured in the same manner as the average particle diameter of the filler (F) described below.

The shape of the aromatic sulfinic acids and salts thereof dispersed as powder is not particularly limited, and may be any of various shapes such as spherical, needle-shape, plate-like, and crushed-shape. Fine powders of aromatic sulfinic acid salts can be prepared using known methods such as pulverization and freeze drying.

In view of working time margin and adhesive properties, the content of aromatic sulfinic acids and salts thereof is preferably 0.1 to 5 parts by mass, more preferably 0.2 to 4 parts by mass, even more preferably 0.5 to 3 parts by mass relative to total 100 parts by mass of the polymerizable monomer (B) in an adhesive composition of the present invention. A content of less than 0.1 parts by mass may cause a decrease in adhesive properties, whereas a content of more than 5 parts by mass may result in shorter working time margins.

Examples of the benzotriazole compound and/or the benzoimidazole compound include compounds represented by the general formula (2) below, and compounds represented by general formula (3). The benzotriazole compounds and benzoimidazole compounds may be used alone, or two or more thereof may be used in combination.

Benzotriazole compounds and benzoimidazole compounds having no substituent at the 2-position, such as compounds represented by general formula (2) and compounds represented by general formula (3), have absorption peaks at wavelengths (for example, 300 nm or less) away from the visible light region. Therefore, unlike benzotriazole compounds having a substituent at the 2-position (for example, 2-(3-tert-butyl-2-hydroxy-5-methylphenyl)-5-chloro-2H-benzotriazole; also referred to as TN326), these compounds exhibit less extended conjugation within the compound, and the peak wavelength does not significantly shift toward the longer wavelength side of the photoabsorption spectrum. In addition, when a catalyst metal (for example, such as copper compounds) is present within the system and the compound coordinates to the catalyst metal as a ligand, the peak wavelength does not greatly shift even when charge transfer between the metal and the ligand causes a shift toward the longer wavelength side of the photoabsorption spectrum, making it possible to reduce the influence of shifting on discoloration.

Consequently, when incorporated as chemical polymerization accelerators (D), compounds represented by general formula (2) and compounds represented by general formula (3) can promote chemical polymerization without impairing the effect of ultraviolet absorber (A).

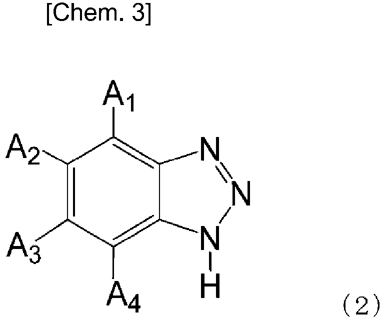

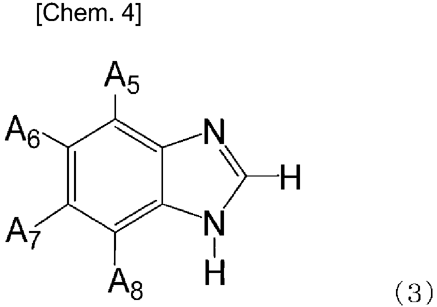

In the general formulae (2) and (3), A₁ to A₈ each independently represent a hydrogen atom, a hydroxyl group, an alkyl group, an aryl group, an alkoxy group, an alkenyl group, an aralkyl group, or a halogen atom.

The alkyl groups represented by A₁ to A₈ may be linear, branched, or cyclic, and have preferably 1 to 10 carbon atoms (3 to 10 carbon atoms for branched and cyclic alkyl groups). Specific examples of the alkyl groups include methyl groups, ethyl groups, n-propyl groups, isopropyl groups, cyclopropyl groups, n-butyl groups, isobutyl groups, sec-butyl groups, tert-butyl groups, cyclobutyl groups, n-pentyl groups, isopentyl groups, neopentyl groups, tert-pentyl groups, cyclopentyl groups, n-hexyl groups, isohexyl groups, cyclohexyl groups, n-heptyl groups, cycloheptanyl groups, n-octyl groups, 2-ethylhexyl groups, cyclooctyl groups, n-nonyl groups, cyclononyl groups, and n-decyl groups. Particularly preferred among these are methyl groups and ethyl groups.

The aryl groups represented by A₁ to A₈ have preferably 6 to 14 carbon atoms. Examples include phenyl groups, naphthyl groups, and anthryl groups.

The alkoxy groups represented by A₁ to A₈ may be linear, branched, or cyclic, and have preferably 1 to 8 carbon atoms. Specific examples include methoxy groups, ethoxy groups, n-propoxy groups, isopropoxy groups, n-butoxy groups, tert-butoxy groups, n-hexyloxy groups, cyclohexyloxy groups, n-octyloxy groups, and 2-ethylhexyloxy groups.

The alkenyl groups represented by A₁ to A₈ may be linear, branched, or cyclic, and have preferably 1 to 6 carbon atoms. Specific examples include vinyl groups, allyl groups, methyl vinyl groups, propenyl groups, butenyl groups, pentenyl groups, hexenyl groups, cyclopropenyl groups, cyclobutenyl groups, cyclopentenyl groups, and cyclohexenyl groups.

Examples of the aralkyl groups represented by A₁ to A₈ include alkyl groups (particularly, C1 to C10 alkyl groups) substituted with aryl groups (particularly, C6 to C10 aryl groups). Specific examples include benzyl groups.

Examples of the halogen atoms represented by A₁ to A₈ include chlorine atoms, bromine atoms, and iodine atoms.

Preferred as A₁ to A₈ are hydrogen atoms or methyl groups.

Specific examples of the benzotriazole compounds and benzoimidazole compounds include 1H-benzotriazole (hereinafter, also referred to by the abbreviation "BTA"), 5-methyl-1H-benzotriazole, 5,6-dimethyl-1H-benzotriazole, benzoimidazole, 5-methylbenzoimidazole, and 5,6-dimethylbenzoimidazole. Among these, 1H-benzotriazole and 5-methyl-1H-benzotriazole are preferred in view of their small conjugated systems, which help more effectively reduce color change in the composition, and thus prevent color change during prolonged storage and provide even superior long-term color stability.

Examples of bromides include zinc bromide, potassium bromide, sodium bromide, calcium bromide, indium bromide, ammonium bromide, and tetrabutylammonium bromide. Particularly preferred are zinc bromide, ammonium bromide, and tetrabutylammonium bromide.

Examples of borate compounds include arylborate compounds having 1 to 4 aryl groups per molecule (for example, such as tetraphenylboron, and tetrakis(p-chlorophenyl)boron), and salts of these.

Examples of barbituric acid compounds include barbituric acid, 5-butyl barbituric acid, 1,3,5-trimethyl barbituric acid, 1-cyclohexyl-5-ethyl barbituric acid, 1-benzyl-5-phenyl barbituric acid, and salts of these.

Examples of ascorbic acid compounds include salts, esters, and ethers of ascorbic acid. Preferred are salts and esters of ascorbic acid.

Examples of salts of ascorbic acid include sodium L-ascorbate, calcium L-ascorbate, potassium ascorbate, and stereoisomers of these (for example, such as sodium isoascorbate). Preferred is sodium L-ascorbate.

Examples of esters of ascorbic acid include compounds formed by reacting one or more hydroxyl groups of ascorbic acid with a carboxylic acid. Preferred examples of the carboxylic acid include C6 to C30 saturated or unsaturated fatty acids, such as caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid, and docosahexaenoic acid. The fatty acids have preferably 10 to 28 carbon atoms, more preferably 12 to 26 carbon atoms, even more preferably 14 to 24 carbon atoms. Particularly preferred for use are esters of stearic acid and ascorbic acid, and esters of palmitic acid and ascorbic acid (ascorbyl palmitate).

Examples of ethers of ascorbic acid include ethyl ascorbic ether, and cetyl ascorbic ether.

Examples of sulfur-containing reducing inorganic compounds include sulfites, bisulfites, pyrosulfites, thiosulfates, thionates, and dithionites. Preferred are sulfites and bisulfites.

Specific examples of sulfur-containing reducing inorganic compounds include sodium sulfite, potassium sulfite, calcium sulfite, ammonium sulfite, sodium bisulfite, and potassium bisulfite.

Preferably, the sulfur-containing reducing inorganic compound is at least partially dispersed in powder form within the composition. With the sulfur-containing reducing inorganic compound being dispersed in powder form, it is possible to provide a longer working time margin for a two-pack type dental composition of the present invention, and, when the composition is applied to the tooth structure, the sulfur-containing reducing inorganic compound dissolves in the water on the surface of the tooth structure, making it possible to more greatly enhance polymerization both at the bonding interface and within the resin-impregnated layer. When the sulfur-containing reducing inorganic compound is dispersed as powder, it is preferable that the sulfur-containing reducing inorganic compound have a solubility in water of 1 mg/100 mL or more at ordinary temperature (25°C). A solubility of 1 mg/100 mL or more facilitates sufficient dissolution of the sulfur-containing reducing inorganic compound in the water on tooth structure at the bonding interface in applications where a two-pack type dental composition of the present invention is applied to tooth structure, facilitating the effect of dispersing as powder. In view of resistance to settling, the sulfur-containing reducing inorganic compound has an average particle diameter of preferably 500 µm or less, more preferably 100 µm or less, even more preferably 50 µm or less. In view of preventing excessive increase in the specific surface area of the powder and facilitating in maintaining good ease of handling for the two-pack type dental composition, the average particle diameter is preferably 0.01 µm or more. That is, when dispersed as powder, the sulfur-containing reducing inorganic compound has an average particle diameter ranging from preferably 0.01 to 500 µm, more preferably from 0.01 to 100 µm. The average particle diameter can be measured in the same manner as the average particle diameter of the filler (F) described below.

The shape of the sulfur-containing reducing inorganic compound dispersed as powder is not particularly limited, and may be any of various shapes such as spherical, needle-shape, plate-like, and crushed-shape. Fine powders of sulfur-containing reducing inorganic compounds can be prepared using known methods such as pulverization and freeze drying.

### [Photopolymerization Initiator (E)]

In order to provide a two-pack type dental composition of the present invention as a dual-cure composition whereby polymerization can also be initiated by photoirradiation, a two-pack type dental composition of the present invention may comprise a known photopolymerization initiator as photopolymerization initiator (E), in addition to chemical polymerization initiator (C).

A certain preferred embodiment is, for example, a two-pack type dental composition that additionally comprises a photopolymerization initiator (E) in at least one of a first agent or a second agent.

Another certain preferred embodiment is, for example, a two-pack type dental composition that additionally comprises a photopolymerization initiator (E) in both a first agent and a second agent.

The photopolymerization initiator (E) may be incorporated in the same agent containing the ultraviolet absorber (A), or may be incorporated separately from the ultraviolet absorber (A).

The photopolymerization initiator (E) is not particularly limited, and any known photopolymerization initiators can be used without restrictions. Examples of known photopolymerization initiators include α-diketones, ketals, thioxanthones, (bis)acylphosphine oxides, α-aminoacetophenones, and amines.

The photopolymerization initiator (E) may be used alone, or two or more thereof may be used in combination.

Examples of α-diketones include dl-camphorquinone (commonly known as CQ), benzil, and 2,3-pentanedione.

Examples of ketals include benzyl dimethyl ketal, and benzyl diethyl ketal.

Examples of thioxanthones include 2-chlorothioxanthone, and 2,4-diethylthioxanthone.

Examples of (bis)acylphosphine oxides include 2,4,6-trimethylbenzoyldiphenylphosphine oxide, bis(2,4,6-trimethylbenzoyl)phenylphosphine oxide, dibenzoylphenylphosphine oxide, bis(2,6-dimethoxybenzoyl)phenylphosphine oxide, tris(2,4-dimethylbenzoyl)phosphine oxide, tris(2-methoxybenzoyl)phosphine oxide, 2,6-dimethoxybenzoyldiphenylphosphine oxide, 2,6-dichlorobenzoyldiphenylphosphine oxide, 2,3,5,6-tetramethylbenzoyldiphenylphosphine oxide, benzoyl-bis(2,6-dimethylphenyl)phosphine oxide, 2,4,6-trimethylbenzoylethoxyphenylphosphine oxide, and the water-soluble acylphosphine oxide compounds disclosed in JP H03-57916 B.

Examples of α-aminoacetophenones include 2-benzyl-2-dimethylamino-1-(4-morpholinophenyl)-1-butanone, 2-benzyl-2-diethylamino-1-(4-morpholinophenyl)-1-butanone, 2-benzyl-2-dimethylamino-1-(4-morpholinophenyl)-1-propanone, 2-benzyl-2-diethylamino-1-(4-morpholinophenyl)-1-propanone, 2-benzyl-2-dimethylamino-1-(4-morpholinophenyl)-1-pentanone, and 2-benzyl-2-diethylamino-1-(4-morpholinophenyl)-1-pentanone.

Examples of amines include aromatic amines.

Examples of aromatic amines include aromatic amines other than aromatic amines having no electron withdrawing group on the aromatic ring serving as chemical polymerization accelerator (D). Examples of such aromatic amines include tertiary aromatic amines having an electron withdrawing group on the aromatic ring, such as n-butoxyethyl 4-(N,N-dimethylamino)benzoate, (2-methacryloyloxy)ethyl 4-(N,N-dimethylamino)benzoate, ethyl 4-(N,N-dimethylamino)benzoate, butyl 4-(N,N-dimethylamino)benzoate, and 4-(N,N-dimethylamino)benzophenone.

In view of the ability to impart superior curability to the two-pack type dental composition, preferred is at least one selected from the group consisting of ethyl 4-(N,N-dimethylamino)benzoate, n-butoxyethyl 4-(N,N-dimethylamino)benzoate, and 4-(N,N-dimethylamino)benzophenone.

In view of properties such as curability, preferred as photopolymerization initiator (E) are α-diketones and acylphosphine oxides, more preferably α-diketones, even more preferably dl-camphorquinone.

In view of curability and storage stability, the content of photopolymerization initiator (E) is preferably 0.005 to 10 parts by mass, more preferably 0.01 to 5 parts by mass, even more preferably 0.1 to 3 parts by mass relative to total 100 parts by mass of polymerizable monomer (B).

### [Filler (F)]

Preferably, a two-pack type dental composition of the present invention further comprises a filler (F).

The filler (F) may be any filler, provided that it does not hinder the effectiveness of the present invention. Examples include inorganic fillers, organic fillers, and composite fillers of inorganic fillers and organic fillers.

The filler (F) may be used alone, or two or more thereof may be used in combination.

Examples of inorganic fillers include the following:
various types of glass (containing silica as a main component, and, optionally, oxides of elements such as heavy metals, boron, and aluminum), including, for example, glass powders of common compositions, such as fused silica, quartz, soda-lime-silica glass, E glass, C glass, and borosilicate glass (PYREX^{®} glass), and dental glass powders such as barium glass (e.g., GM27884 and 8235 manufactured by SCHOTT, and E-2000 and E-3000 manufactured by ESSTECH), strontium·borosilicate glass (e.g., E-4000 manufactured by ESSTECH), lanthanum glass-ceramic (e.g., GM31684 manufactured by SCHOTT), and fluoroaluminosilicate glass (e.g., GM35429, G018-091, and G018-117 manufactured by SCHOTT); and
various types of ceramics, composite oxides (such as silica-titania, and silica-zirconia), diatomaceous earth, kaolin, clay minerals (such as montmorillonite), activated earth, synthetic zeolite, mica, calcium fluoride, ytterbium fluoride, yttrium fluoride, core-shell structured calcium fluoride with silica coating, core-shell structured ytterbium fluoride with silica coating, core-shell structured yttrium fluoride with silica coating, calcium phosphate, barium sulfate, zirconium dioxide, titanium dioxide, hydroxyapatite, core-shell structured calcium phosphate with silica coating, core-shell structured barium sulfate with silica coating, core-shell structured zirconium dioxide with silica coating, core-shell structured titanium dioxide with silica coating, and core-shell structured hydroxyapatite with silica coating.

In view of strength and other properties, preferred among these are various types of glass, composite oxides (such as silica-titania, and silica-zirconia), core-shell structured calcium fluoride with silica coating, core-shell structured ytterbium fluoride with silica coating, core-shell structured yttrium fluoride with silica coating, core-shell structured calcium phosphate with silica coating, core-shell structured barium sulfate with silica coating, core-shell structured zirconium dioxide with silica coating, core-shell structured titanium dioxide with silica coating, and core-shell structured hydroxyapatite with silica coating.

In view of adhesive properties and handling properties, preferred for use is particulate silica with an average primary particle diameter of 0.001 to 10 µm.

Examples of commercially available products include Aerosil^{®} OX50, Aerosil^{®} 50, Aerosil^{®} 200, Aerosil^{®} 380, Aerosil^{®} R972, and Aerosil^{®} 130 (all manufactured by Nippon Aerosil Co., Ltd. under these trade names).

In the present invention, the average particle diameter of inorganic fillers means the average particle diameter before surface treatment when the inorganic filler is surface treated as in the descriptions below.

Examples of organic fillers include polymethyl methacrylate, polyethyl methacrylate, a methyl methacrylate-ethyl methacrylate copolymer, crosslinked polymethyl methacrylate, crosslinked polyethyl methacrylate, polyamide, polyvinyl chloride, polystyrene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer.

Examples of inorganic-organic composite fillers include those with an inorganic filler dispersed in an organic filler, and inorganic-organic composite fillers where the inorganic filler is coated with various polymers.

In this specification, the average particle diameter of filler (F) can be determined by a laser diffraction scattering method or electron microscopy of particles. Specifically, a laser diffraction scattering method is more convenient for the measurement of particles with a particle size of 0.1 µm or more, whereas electron microscopy is a more convenient method of particle size measurement for ultrafine particles of less than 0.1 µm. Here, 0.1 µm is a measured value by a laser diffraction scattering method.

As a specific example of a laser diffraction scattering method, the particle size may be measured by volume using, for example, a laser diffraction particle size distribution analyzer (SALD-2300, manufactured by Shimadzu Corporation) with a 0.2% sodium hexametaphosphate aqueous solution used as dispersion medium.

In electron microscopy, for example, filler particles may be photographed with a scanning electron microscope (Model S-4000, manufactured by Hitachi, Ltd.), and the size of particles (at least 200 particles) observed in a unit field of the captured image may be measured using image-analyzing particle size distribution measurement software (Mac-View, manufactured by Mountech Co., Ltd.). Here, the particle diameter is determined as an arithmetic mean value of the maximum and minimum lengths of particles, and the average particle diameter is calculated from the number of particles and the particle diameter.

In order to improve curability, mechanical strength, and handling properties, the filler (F) may be used after surface treatment with a known surface treatment agent such as a silane coupling agent.

Examples of the surface treatment agent include silane coupling agents such as vinyltrimethoxysilane, vinyltriethoxysilane, vinyltrichlorosilane, vinyltri(β-methoxyethoxy)silane, γ-methacryloyloxypropyltrimethoxysilane, γ-glycidoxypropyltrimethoxysilane, γ-mercaptopropyltrimethoxysilane, and γ-aminopropyltriethoxysilane.

A two-pack type dental composition of the present invention may additionally comprise a silane coupling agent other than a silane coupling agent serving as a surface treatment agent for the filler (F).

The silane coupling agent may be used alone, or two or more thereof may be used in combination.

The silane coupling agent may be a known silane coupling agent, without any restrictions.

Specific examples of the silane coupling agent include vinyltrimethoxysilane, vinyltriethoxysilane, vinyltrichlorosilane, vinyltri(β-methoxyethoxy)silane, γ-methacryloyloxypropyltrimethoxysilane, γ-glycidoxypropyltrimethoxysilane, γ-mercaptopropyltrimethoxysilane, γ-aminopropyltriethoxysilane, 5-(meth)acryloyloxypentyltrimethoxysilane, 6-(meth)acryloyloxyhexyltrimethoxysilane, 7-(meth)acryloyloxyheptyltrimethoxysilane, 8-(meth)acryloyloxyoctyltrimethoxysilane, 9-(meth)acryloyloxynonyltrimethoxysilane, 10-(meth)acryloyloxydecyltrimethoxysilane, 11-(meth)acryloyloxyundecyltrimethoxysilane, 8-(meth)acryloyloxyoctylmethyldimethoxysilane, 10-(meth)acryloyloxydecylmethyldimethoxysilane, 11-(meth)acryloyloxyundecylmethyldimethoxysilane, and (meth)acryloyloxymethylphenethyltrimethoxysilane.

In view of adhesive properties, the content of the silane coupling agent is preferably 0.1 to 10.0 mass%, more preferably 0.5 to 9.0 mass%, even more preferably 1.0 to 8.0 mass%, particularly preferably 1.2 to 7.0 mass% in 100 mass% of the total mass of a two-pack type dental composition of the present invention.

To enhance photocurability, the photopolymerization initiator (E) may be used with a polymerization accelerator such as aldehydes, thiol compounds, or triazine compounds substituted with trihalomethyl groups.

However, a certain preferred embodiment is, for example, a two-pack type dental composition that comprises an ultraviolet absorber (A) without a triazine compound (for example, a triazine compound substituted with a trihalomethyl group).

Examples of aldehydes include terephthalaldehyde and benzaldehyde derivatives. Examples of benzaldehyde derivatives include dimethylaminobenzaldehyde, p-methoxybenzaldehyde, p-ethoxybenzaldehyde, and p-n-octyloxybenzaldehyde.

Examples of thiol compounds include 3-mercaptopropyltrimethoxysilane, 2-mercaptobenzoxazole, decanethiol, and thiobenzoic acid.

The polymerization accelerator may be used alone, or two or more thereof may be used in combination.

A two-pack type dental composition of the present invention may comprise a fluoride ion-releasing substance to impart acid resistance to tooth structure. The fluoride ion-releasing substance may be used alone, or two or more thereof may be used in combination.

Examples of the fluoride ion-releasing substance include fluoride ion-releasing polymers such as copolymers of methyl methacrylate and methacryloyl fluoride; fluoride ion-releasing substances such as cetylamine hydrofluoride; and inorganic fillers such as fluoroaluminosilicate glass, sodium fluoride, potassium fluoride, sodium monofluorophosphate, lithium fluoride, and ytterbium fluoride.

A two-pack type dental composition of the present invention may comprise additives such as a cross-linking agent, a silanol condensation catalyst, a stabilizer (polymerization inhibitor), a colorant (dye, pigment), and a fluorescent agent. The additives may be used alone, or two or more thereof may be used in combination.

The cross-linking agent and the silanol condensation catalyst may be those described in WO2019/004391.

Examples of the polymerization inhibitor include hydroquinone, hydroquinone monomethyl ether, dibutyl hydroquinone, dibutyl hydroquinone monomethyl ether, t-butyl catechol, 2-t-butyl-4,6-dimethylphenol, 2,6-di-t-butyl-4-methylphenol, and 2,6-di-t-butylphenol.

It is also possible to comprise anti-microbial substances such as cetylpyridinium chloride, benzalkonium chloride, (meth)acryloyloxydodecylpyridinium bromide, (meth)acryloyloxyhexadecylpyridinium chloride, (meth)acryloyloxydecylammonium chloride, and triclosan. The anti-microbial substances may be used alone, or two or more thereof may be used in combination.

In a certain preferred embodiment, a two-pack type dental composition of the present invention is composed of a first agent and a second agent, and the first agent comprises preferably an ultraviolet absorber (A), a polymerizable monomer (B) (for example, a polymerizable monomer having no acidic group (B-2)), and a chemical polymerization accelerator (D), more preferably an ultraviolet absorber (A), a polymerizable monomer (B) (for example, a polymerizable monomer having no acidic group (B-2)), a chemical polymerization accelerator (D), and a filler (F).

In the case of dual-cure polymerization, it is preferable that the first agent further comprises a photopolymerization initiator (E).

The second agent comprises preferably a polymerizable monomer (B) (for example, a polymerizable monomer having no acidic group (B-2)), and a chemical polymerization initiator (C), more preferably a polymerizable monomer (B) (for example, a polymerizable monomer having no acidic group (B-2)), a chemical polymerization initiator (C), and a chemical polymerization accelerator (D), even more preferably a polymerizable monomer (B) (for example, a polymerizable monomer having no acidic group (B-2)), a chemical polymerization initiator (C), a chemical polymerization accelerator (D), and a filler (F).

When the adhesive properties of a two-pack type dental composition of the present invention should be increased, it is particularly preferable that the polymerizable monomer (B) in the second agent comprises a polymerizable monomer having an acidic group (B-1), and a polymerizable monomer having no acidic group (B-2).

In another certain preferred embodiment, a two-pack type dental composition of the present invention is composed of a first agent and a second agent, and the first agent comprises preferably a polymerizable monomer (B) (for example, a polymerizable monomer having no acidic group (B-2), and a chemical polymerization accelerator (D), more preferably a polymerizable monomer (B) (for example, a polymerizable monomer having no acidic group (B-2), a chemical polymerization accelerator (D), and a filler (F).

The second agent comprises preferably an ultraviolet absorber (A), a polymerizable monomer (B) (for example, a polymerizable monomer having no acidic group (B-2), and a chemical polymerization initiator (C), more preferably an ultraviolet absorber (A), a polymerizable monomer (B) (for example, a polymerizable monomer having no acidic group (B-2), a chemical polymerization initiator (C), and a chemical polymerization accelerator (D), even more preferably an ultraviolet absorber (A), a polymerizable monomer (B) (for example, a polymerizable monomer having no acidic group (B-2), a chemical polymerization initiator (C), a chemical polymerization accelerator (D), and a filler (F).

In the case of dual-cure polymerization, it is preferable that the second agent further comprises a photopolymerization initiator (E).

When the adhesive properties of a two-pack type dental composition of the present invention should be increased, it is particularly preferable that the polymerizable monomer (B) in the second agent comprises a polymerizable monomer having an acidic group (B-1), and a polymerizable monomer having no acidic group (B-2).

Preferably, a two-pack type dental composition of the present invention does not comprise a solvent (water, organic solvent).

In a certain preferred embodiment, the content of the solvent (water, organic solvent) in a two-pack type dental composition of the present invention is preferably less than 5.0 mass%, more preferably less than 1.0 mass%, even more preferably less than 0.5 mass%, particularly preferably less than 0.1 mass%, most preferably less than 0.01 mass%.

A two-pack type dental composition of the present invention can be produced, for example, by mixing components other than the powder components (e.g., filler (F)) to obtain a solution, and subsequently adding the powder components.

The present invention encompasses embodiments combining all or part of the foregoing features in various forms, provided that the present invention can exhibit its effects with such combinations within the technical idea of the present invention.

### EXAMPLES

The present invention is described below in greater detail through Examples. It is to be noted, however, that the present invention is in no way limited by the following Examples, and various modifications may be made by a person with ordinary skill in the art within the technical idea of the present invention. The following abbreviations and symbols are used.

### [Ultraviolet Absorber (A)]

T1: bisethylhexyloxyphenol methoxyphenyl triazine
T2: 2-[4,6-bis(biphenyl-4-yl)-1,3,5-triazin-2-yl]-5-[(2-ethylhexyl)oxy]phenol
T3: 2,4,6-tris[4-(hexyloxy)-2-hydroxy-3-methylphenyl]-1,3,5-triazine
T4: 2,4-bis(2,4-dimethylphenyl)-6-(2-hydroxy-4-n-octyloxyphenyl)-1,3,5-triazine

### [Ultraviolet Absorbers Other Than Ultraviolet Absorber (A)]

B1: 2-(3-tert-butyl-2-hydroxy-5-methylphenyl)-5-chloro-2H-benzotriazole
B2: 2-(2H-benzotriazol-2-yl)-4-methyl-6-dodecylphenol
O2: 2-ethylhexyl 4-methoxycinnamate

### [Polymerizable Monomer Having Acidic Group (B-1)]

MDP: 10-methacryloyloxydecyl dihydrogen phosphate

### [Polymerizable Monomer Having no Acidic Group (B-2)]

UDMA: 2,2,4-trimethylhexamethylenebis(2-carbamoyloxyethyl) dimethacrylate
HEMA: 2-hydroxyethyl methacrylate
Bis-GMA: 2,2-bis[4-(2-hydroxy-3-methacryloyloxypropoxy)phenyl]propane
3G: triethylene glycol dimethacrylate
NPG: neopentyl glycol dimethacrylate
D-2.6E: 2,2-bis(4-methacryloyloxypolyethoxyphenyl)propane (with an average of 2.6 moles of ethoxy group added)
#801: 1,2-bis(3-methacryloyloxy-2-hydroxypropoxy)ethane
POBMA: m-phenoxybenzyl methacrylate

### [Chemical Polymerization Initiator (C)]

BPB: t-butyl peroxybenzoate
KPS: potassium peroxydisulfate
BPO: benzoyl peroxide
THP: 1,1,3,3-tetramethylbutyl hydroperoxide

### [Chemical Polymerization Accelerator (D)]

TPSS: sodium 2,4,6-triisopropylbenzenesulfinate
DEPT: N,N-bis(2-hydroxyethyl)-p-toluidine
BTA: 1H-benzotriazole
NAA: sodium sulfite
CuA: copper(II) acetate
PyTU: 1-(2-pyridyl)-2-thiourea
VOAA: vanadyl(IV) acetylacetonate
NMDEA: N-methyldiethanolamine

### [Photopolymerization Initiator (E)]

CQ: dl-camphorquinone
DABE: ethyl 4-(N,N-dimethylamino)benzoate
TPO: 2,4,6-trimethylbenzoyldiphenylphosphine oxide

### [Filler (F)]

Ba1: silane-treated barium glass powder:
A barium glass (Raysorb E-3000 manufactured by Esstech under this trade name) was pulverized with a ball mill to obtain a barium glass powder having an average particle diameter of approximately 2.4 µm. The barium glass powder was then surface-treated with 3 parts by mass of γ-methacryloyloxypropyltrimethoxysilane relative to 100 parts by mass of the powder, using an ordinary method. This yielded a silane-treated barium glass powder.

Ba2: silane-treated barium glass powder:
A barium glass (Raysorb E-3000 manufactured by Esstech under this trade name) was pulverized with a ball mill to obtain a barium glass powder having an average particle diameter of approximately 2.4 µm. The barium glass powder was then surface-treated with 3 parts by mass of γ-methacryloyloxypropyltrimethoxysilane relative to 100 parts by mass of the powder, using an ordinary method. This yielded a silane-treated barium glass powder.
Al₂O₃: AEROXIDE^{®} Alu C manufactured by Nippon Aerosil Co., Ltd. under this trade name; average particle diameter: 13 nm
SiO₂: particulate silica Aerosil^{®} R972 manufactured by Nippon Aerosil Co., Ltd.; average particle diameter: 16 nm

Ba3: A three-neck flask was charged with 100 g of a barium glass (8235 K4 manufactured by SCHOTT; average particle diameter: 7 µm), 6 g of γ-methacryloyloxypropyltrimethoxysilane, and 200 mL of a 0.3 mass% acetic acid aqueous solution. These were then stirred at room temperature for 2 hours. After freeze drying to remove water, the resulting material was subjected to heat treatment at 80°C for 5 hours to yield a silane-treated barium glass.

Ba4: A three-neck flask was charged with 100 g of a barium glass (8235 UF0.7 grade manufactured by SCHOTT; average particle diameter: 0.7 µm), 6 g of γ-methacryloyloxypropyltrimethoxysilane, and 200 mL of a 0.3 mass% acetic acid aqueous solution. These were then stirred at room temperature for 2 hours. After freeze drying to remove water, the resulting material was subjected to heat treatment at 80°C for 5 hours to yield a silane-treated barium glass.

G018-090UF2.0: A three-neck flask was charged with 100 g of a fluoroaluminosilicate glass (G018-090, grade UF2.0, manufactured by SCHOTT under this trade name; average particle diameter: 2.0 µm), 6 g of γ-methacryloyloxypropyltrimethoxysilane, and 200 mL of a 0.3 mass% acetic acid aqueous solution. These were then stirred at room temperature for 2 hours. After freeze drying to remove water, the resulting material was subjected to heat treatment at 80°C for 5 hours to yield a silane-treated fluoroaluminosilicate glass.

Ar380: hydrophilic fumed silica Aerosil^{®} 380 manufactured by Nippon Aerosil Co., Ltd.; average primary particle diameter: 0.007 µm

### [Others]

γMPS: γ-methacryloyloxypropyltrimethoxysilane (silane coupling agent)
BHT: 2,6-di-t-butyl-4-methylphenol (polymerization inhibitor)
TCT: 2,4,6-tris(trichloromethyl)-s-triazine

### [Examples 1-1 to 1-8, 2-1 to 2-4, 3-1 and 3-2, 4-1 and 4-2, and Comparative Examples 1-1 to 1-7, 2-1 to 2-5, 3-1 to 3-3, and 4-1 to 4-3]

First and second agents of the formulations shown in Tables 2 to 6 were prepared.

To prepare the first agent, the components other than the powder components (filler, TPSS, NAA) were blended and stirred to form a homogeneous solution. The powder components were then kneaded into the solution and the mixture was degassed. The powder components in the first agent remained dispersed in powder form.

To prepare the second agent, the components other than the powder components (filler and KPS) were blended and stirred to form a homogeneous solution. The powder components were then kneaded into the solution and the mixture was degassed. The powder components in the second agent remained dispersed in powder form. The two agents were separately filled into a double syringe (a 5 mL double syringe manufactured by Mixpac). After setting the plunger, a mixing tip (manufactured by Mixpac) was attached to the tip of the double syringe, and the two agents were auto-mixed at a volume ratio of 1:1. The resulting mixture was then used as a two-pack type dental composition for evaluation. The tests were conducted according to the methods described below. The results are presented in Tables 2 to 6. Table 1 shows the properties of the ultraviolet absorber (A) and the ultraviolet absorbers other than ultraviolet absorber (A) used.

### [Measurement of Maximum Absorption Wavelength]

A 3 × 10⁻⁵ mol/L solution was prepared using ethyl acetate as the solvent. This solution was then measured for maximum absorption wavelength over a wavelength range of 250 nm to 500 nm, using a spectrophotometer (UH5200, manufactured by Hitachi High-Tech Corporation under this trade name) with a quartz cell having an optical path length of 10 mm (arithmetic mean value for n = 2).

### [Evaluation of Color Stability]

For the two-pack type dental composition of each Example and Comparative Example, the first and second agents were thoroughly mixed for 10 seconds at a volume ratio of 1:1 to prepare a paste (two-pack type dental composition). A mold, 1 mm thick and having a through hole with a diameter of 15 mm, was placed on a PET sheet laid on a glass slide, and the paste was immediately filled into the mold, which was then covered with another PET sheet and glass slide with pressure applied from above. Subsequently, the paste was irradiated with light from both sides, 5 points on each side, 10 seconds each, using a dental polymerization photoirradiator (PenCure 2000 manufactured by J. Morita Corp. under this trade name), ensuring that the entire paste was exposed to light. This resulted in a cured product to be used as a test specimen.

The cured product was placed in a desiccator and stored in the dark at 37°C for 24 hours. The color was then measured using a spectrocolorimeter (SE6000 manufactured by Nippon Denshoku Industries Co., Ltd. under this trade name), and this value was taken as the chromaticity before irradiation.

Subsequently, the cured product was irradiated with light for 24 hours in 37°C water at an intensity of 500 to 550 W/m² and 150,000 lx (lux), using a xenon accelerated weathering tester (Solarbox 1500e manufactured by Bunkoukeiki Co., Ltd. under this trade name). After irradiation, the cured product was measured for color using the spectrocolorimeter, and the measured value was taken as the chromaticity after irradiation. The color change ΔE* was then calculated from the chromaticity values before and after irradiation (arithmetic mean value for n = 2).

The color change ΔE* can be calculated using the equations below. A smaller value indicates a smaller color change and higher color stability to sunlight. $ΔE * = {\left\{{\left(ΔL*\right)}^{2}+{\left(Δa*\right)}^{2}+{\left(Δb*\right)}^{2}\right\}}^{1 / 2} ;$ $ΔL * = L 1 * - L 2 * ;$ $Δa * = a 1 * - a 2 * ;$ $Δb * = b 1 * - b 2 * :$

Here, L1* represents the lightness index before irradiation, a1* and b1* represent the chromaticness indices before irradiation, L2* represents the lightness index after irradiation, and a2* and b2* represent the chromaticness indices after irradiation.

In addition to evaluating the cured product of the composition produced by mixing the first and second agents immediately after preparation, the evaluation was also conducted by simulating prolonged storage conditions using a cured product of a composition produced by mixing the first and second agents after 2 weeks of storage at 60°C in the double syringe. In Tables 2 to 6, the first evaluation is indicated by "ΔE* (initial)", and the second evaluation is indicated by "ΔE* (after temperature acceleration)".

### [Unpolymerized Thickness (Photocure)]

A polyester film was laid on a glass slide, and a 1 mm-thick Teflon^{®} mold with a hole (Ø = 5 mm) was placed on the film. The two-pack type dental composition prepared above was then filled into the mold, and its mass was measured as (X) [g].

The two-pack type dental composition was irradiated with light for 10 seconds from above, using a dental polymerization photoirradiator PenCure 2000 (manufactured by J. Morita Corp. under this trade name). Subsequently, the uncured portion was removed by wiping it off with a JK Wiper^{®} (manufactured by Nippon Paper Crecia Co., Ltd.), and the remaining mass was measured as (Y) [g]. The unpolymerized thickness was calculated using the following equation (arithmetic mean value for n = 5). 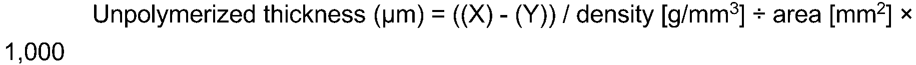 Unpolymerized thickness (µm) = ((X) - (Y)) / density [g/mm3] ÷ area [mm2] × 1,000

### [Unpolymerized Thickness (Chemical Cure)]

A polyester film was laid on a glass slide, and a 1 mm-thick Teflon^{®} mold with a hole (Ø = 5 mm) was placed on the film. The two-pack type dental composition prepared above was then filled into the mold, and its mass was measured as (X) [g]. After curing the two-pack type dental composition at a constant temperature of 37°C for 10 minutes, the uncured portion was removed by wiping it off with a JK Wiper^{®} (manufactured by Nippon Paper Crecia Co., Ltd.), and the remaining mass was measured as (Y) [g].

The unpolymerized thickness was calculated using the following equation (arithmetic mean value for n = 5). 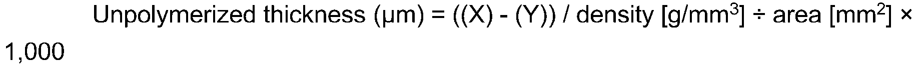 Unpolymerized thickness (µm) = ((X) - (Y)) / density [g/mm3] ÷ area [mm2] × 1,000

**[Table 1]**

| | | Presence or absence of triazine skeleton | Maximum absorption wavelength (nm) |
|---|---|---|---|
| Ultraviolet absorber (A) | T1 | Present | 310 to 320, 350 to 360 |
| | T2 | Present | 315 to 325, 345 to 355 |
| | T3 | Present | 355 to 365 |
| | T4 | Present | 285 to 295, 335 to 345 |
| Ultraviolet absorbers other than ultraviolet absorber (A) | B1 | Absent | 305 to 315, 350 to 360 |
| | B2 | Absent | 345 to 355 |
| | O1 | Absent | 305 to 315 |

**[Table 2]**

| | Components (mass%) | | Ex. 1-1 | Ex. 1-2 | Ex. 1-3 | Ex. 1-4 | Ex. 1-5 | Ex. 1-6 | Ex. 1-7 | Ex. 1-8 |
|---|---|---|---|---|---|---|---|---|---|---|
| | Ultraviolet absorber (A) | T1 | 0.10 | 0.01 | | | | 0.10 | 0.10 | |
| | | T2 | | | 0.10 | | | | | |
| | | T3 | | | | 0.10 | | | | |
| | | T4 | | | | | 0.10 | | | |
| | Ultraviolet absorbers other than ultraviolet absorber (A) | B1 | | | | | | | | |
| | | B2 | | | | | | | | |
| | | O2 | | | | | | | | |
| | Polymerizable monomer having no acidic group (B-2) | UDMA | 15.00 | 15.00 | 15.00 | 15.00 | 15.00 | 15.00 | 15.00 | 15.00 |
| First agent | Chemical polymerization accelerator (D) | TPSS | 0.50 | 0.50 | 0.50 | 0.50 | 0.50 | 0.50 | 0.50 | 0.50 |
| | | DEPT | 0.01 | 0.01 | 0.01 | 0.01 | 0.01 | 0.01 | 0.01 | 0.01 |
| | | BTA | 0.50 | 0.50 | 0.50 | 0.50 | 0.50 | 0.50 | 0.50 | 0.50 |
| | | NAA | 0.50 | 0.50 | 0.50 | 0.50 | 0.50 | 0.50 | 0.50 | 0.50 |
| | Photopolymerization initiator (E) | CQ | 0.01 | 0.01 | 0.01 | 0.01 | 0.01 | 0.01 | 0.01 | |
| | | DABE | 0.01 | 0.01 | 0.01 | 0.01 | 0.01 | 0.01 | | 0.01 |
| | Filler (F) | Ba2 | 24.35 | 24.44 | 24.35 | 24.35 | 24.35 | 24.35 | 24.35 | 24.35 |
| | | Al₂O₃ | 5.00 | 5.00 | 5.00 | 5.00 | 5.00 | 5.00 | 5.00 | 5.00 |
| | Other components | yMPS | 4.00 | 4.00 | 4.00 | 4.00 | 4.00 | 4.00 | 4.00 | 4.00 |
| | | BHT | 0.02 | 0.02 | 0.02 | 0.02 | 0.02 | 0.02 | 0.02 | 0.02 |
| Second agent | Ultraviolet absorber (A) | T1 | | | | | | | | 0.10 |
| | Polymerizable monomer having acidic group (B-1) | MDP | 3.00 | 3.00 | 3.00 | 3.00 | 3.00 | 3.00 | 3.00 | 3.00 |
| | Polymerizable monomer having no acidic group (B-2) | Bis-GMA | 10.00 | 10.00 | 10.00 | 10.00 | 10.00 | 10.00 | 10.00 | 10.00 |
| | | HEMA | 2.00 | 2.00 | 2.00 | 2.00 | 2.00 | 2.00 | 2.00 | 2.00 |
| | | 3G | 2.00 | 2.00 | 2.00 | 2.00 | 2.00 | 2.00 | 2.00 | 2.00 |
| | Chemical polymerization initiator (C) | BPB | 0.25 | 0.25 | 0.25 | 0.25 | 0.25 | 0.25 | 0.25 | 0.25 |
| | | KPS | 0.25 | 0.25 | 0.25 | 0.25 | 0.25 | 0.25 | 0.25 | 0.25 |
| | Chemical polymerization accelerator (D) | CuA | 0.00005 | 0.00005 | 0.00005 | 0.00005 | 0.00005 | 0.00005 | 0.00005 | 0.00005 |
| | Photopolymerization initiator (E) | CQ | | | | | | | | 0.01 |
| | Filler (F) | SiO₂ | 5.00 | 5.00 | 5.00 | 5.00 | 5.00 | 5.00 | 5.00 | 5.00 |
| | | Ba1 | 27.49 | 27.49 | 27.49 | 27.49 | 27.49 | 27.48 | 27.50 | 27.48 |
| | Other components | TCT | | | | | | 0.01 | | 0.01 |
| | | BHT | 0.01 | 0.01 | 0.01 | 0.01 | 0.01 | 0.01 | 0.01 | 0.01 |
| Color change ΔE* (initial) | | | 1.6 | 2.0 | 0.5 | 2.2 | 2.1 | 1.0 | 0.8 | 1.3 |
| Color change ΔE* (after temperature acceleration) | | | 3.2 | 4.0 | 3.5 | 3.8 | 4.0 | 3.0 | 3.0 | 2.5 |
| Thickness of unpolymerized layer (photocure) (µm) | | | 18.2 | 18.3 | 20.6 | 27.3 | 24.6 | 13.2 | - | 13.2 |
| Thickness of unpolymerized layer (chemical cure) (µm) | | | - | - | - | - | - | - | 118.3 | - |

**[Table 3]**

| | Components (mass%) | | Com. Ex. 1-1 | Com. Ex. 1-2 | Com. Ex. 1-3 | Com. Ex. 1-4 | Com. Ex. 1-5 | Com. Ex. 1-6 | Com. Ex. 1-7 |
|---|---|---|---|---|---|---|---|---|---|
| | Ultraviolet absorber (A) | T1 | | | | | | | 0.10 |
| | | T2 | | | | | | | |
| | | T3 | | | | | | | |
| | | T4 | | | | | | | |
| | Ultraviolet absorbers other than ultraviolet absorber (A) | B1 | | 0.10 | 0.01 | | | 0.10 | |
| | | B2 | | | | 0.10 | | | |
| | | O2 | | | | | 0.10 | | |
| | Polymerizable monomer having no acidic group (B-2) | UDMA | 15.00 | 15.00 | 15.00 | 15.00 | 15.00 | 15.00 | 15.00 |
| First agent | Chemical polymerization accelerator (D) | TPSS | 0.50 | 0.50 | 0.50 | 0.50 | 0.50 | 0.50 | |
| | | DEPT | 0.01 | 0.01 | 0.01 | 0.01 | 0.01 | 0.01 | |
| | | BTA | 0.50 | 0.50 | 0.50 | 0.50 | 0.50 | 0.50 | |
| | | NAA | 0.50 | 0.50 | 0.50 | 0.50 | 0.50 | 0.50 | |
| | Photopolymerization initiator (E) | CQ | 0.01 | 0.01 | 0.01 | 0.01 | 0.01 | | |
| | | DABE | 0.01 | 0.01 | 0.01 | 0.01 | 0.01 | | |
| | Filler (F) | Ba2 | 24.45 | 24.35 | 24.44 | 24.35 | 24.35 | 24.37 | 25.88 |
| | | Al₂O₃ | 5.00 | 5.00 | 5.00 | 5.00 | 5.00 | 5.00 | 5.00 |
| | Other components | yMPS | 4.00 | 4.00 | 4.00 | 4.00 | 4.00 | 4.00 | 4.00 |
| | | BHT | 0.02 | 0.02 | 0.02 | 0.02 | 0.02 | 0.02 | 0.02 |
| Second agent | Ultraviolet absorber (A) | T1 | | | | | | | |
| | Polymerizable monomer having acidic group (B-1) | MDP | 3.00 | 3.00 | 3.00 | 3.00 | 3.00 | 3.00 | 3.00 |
| | Polymerizable monomer having no acidic group (B-2) | Bis-GMA | 10.00 | 10.00 | 10.00 | 10.00 | 10.00 | 10.00 | 10.00 |
| | | HEMA | 2.00 | 2.00 | 2.00 | 2.00 | 2.00 | 2.00 | 2.00 |
| | | 3G | 2.00 | 2.00 | 2.00 | 2.00 | 2.00 | 2.00 | 2.00 |
| | Chemical polymerization initiator (C) | BPB | 0.25 | 0.25 | 0.25 | 0.25 | 0.25 | 0.25 | 0.25 |
| | | KPS | 0.25 | 0.25 | 0.25 | 0.25 | 0.25 | 0.25 | 0.25 |
| | Chemical polymerization accelerator (D) | CuA | 0.00050 | 0.00005 | 0.00005 | 0.00005 | 0.00005 | 0.00005 | |
| | Photopolymerization initiator (E) | CQ | | | | | | | |
| | Filler (F) | SiO₂ | 5.00 | 5.00 | 5.00 | 5.00 | 5.00 | 5.00 | 5.00 |
| | | Ba1 | 27.48 | 27.49 | 27.49 | 27.49 | 27.49 | 27.49 | 27.49 |
| | Other components | TCT | 0.01 | | | | | | |
| | | BHT | 0.01 | 0.01 | 0.01 | 0.01 | 0.01 | 0.01 | 0.01 |
| Color change ΔE* (initial) | | | 5.0 | 0.6 | 3.5 | 1.1 | 4.9 | 0.6 | No cure |
| Color change ΔE* (after temperature acceleration) | | | 9.4 | 4.2 | 7.5 | 4.2 | 8.2 | 4.1 | No cure |
| Thickness of unpolymerized layer (photocure) (µm) | | | 16.9 | 28.0 | 27.5 | 28.5 | 30.0 | - | No cure |
| Thickness of unpolymerized layer (chemical cure) (µm) | | | - | - | - | - | - | 141.1 | No cure |

**[Table 4]**

| | Components (mass%) | | Ex. 2-1 | Ex. 2-2 | Ex. 2-3 | Ex. 2-4 | Com. Ex. 2-1 | Com. Ex. 2-2 | Com. Ex. 2-3 | Com. Ex. 2-4 | Com. Ex. 2-5 |
|---|---|---|---|---|---|---|---|---|---|---|---|
| | Ultraviolet absorber (Example) (A) | T1 | 0.15 | 0.05 | | | | | | | |
| | | T2 | | | 0.15 | 0.05 | | | | | |
| | Ultraviolet absorbers other than ultraviolet absorber (A) | B1 | | | | | | 0.15 | 0.05 | | |
| | | B2 | | | | | | | | 0.15 | 0.05 |
| | Polymerizable monomer having no acidic group (B-2) | D-2.6E | 10.00 | 10.00 | 10.00 | 10.00 | 10.00 | 10.00 | 10.00 | 10.00 | 10.00 |
| | | #801 | 3.00 | 3.00 | 3.00 | 3.00 | 3.00 | 3.00 | 3.00 | 3.00 | 3.00 |
| First agent | | 3G | 1.00 | 1.00 | 1.00 | 1.00 | 1.00 | 1.00 | 1.00 | 1.00 | 1.00 |
| | Chemical polymerization accelerator (D) | DEPT | 0.30 | 0.30 | 0.30 | 0.30 | 0.30 | 0.30 | 0.30 | 0.30 | 0.30 |
| | | NAA | 1.00 | 1.00 | 1.00 | 1.00 | 1.00 | 1.00 | 1.00 | 1.00 | 1.00 |
| | Filler (F) | Ba1 | 31.84 | 31.94 | 31.84 | 31.94 | 31.99 | 31.84 | 31.94 | 31.84 | 31.94 |
| | | Al₂O₃ | 2.50 | 2.50 | 2.50 | 2.50 | 2.50 | 2.50 | 2.50 | 2.50 | 2.50 |
| | Photopolymerization initiator (E) | DABE | 0.20 | 0.20 | 0.20 | 0.20 | 0.20 | 0.20 | 0.20 | 0.20 | 0.20 |
| | Other components | BHT | 0.01 | 0.01 | 0.01 | 0.01 | 0.01 | 0.01 | 0.01 | 0.01 | 0.01 |
| Second agent | Polymerizable monomer having acidic group (B-1) | MDP | | | | | | | | | |
| | Polymerizable monomer having no acidic group (B-2) | Bis-GMA | 3.00 | 6.00 | 6.00 | 6.00 | 6.00 | 6.00 | 6.00 | 6.00 | 6.00 |
| | | NPG | 5.00 | 10.50 | 10.50 | 10.50 | 10.50 | 10.50 | 10.50 | 10.50 | 10.50 |
| | | #801 | 3.00 | 3.50 | 3.50 | 3.50 | 3.50 | 3.50 | 3.50 | 3.50 | 3.50 |
| | Chemical polymerization initiator (C) | BPO | 0.50 | 0.38 | 0.38 | 0.38 | 0.38 | 0.38 | 0.38 | 0.38 | 0.38 |
| | Photopolymerization initiator (E) | CQ | 0.20 | 0.25 | 0.25 | 0.25 | 0.25 | 0.25 | 0.25 | 0.25 | 0.25 |
| | | TPO | 0.10 | 0.03 | 0.03 | 0.03 | 0.03 | 0.03 | 0.03 | 0.03 | 0.03 |
| | Filler (F) | SiO₂ | 4.00 | 0.50 | 0.50 | 0.50 | 0.50 | 0.50 | 0.50 | 0.50 | 0.50 |
| | | Ba3 | 34.19 | 66.83 | 66.83 | 66.83 | 66.83 | 66.83 | 66.83 | 66.83 | 66.83 |
| | Other components | BHT | 0.01 | 0.02 | 0.02 | 0.02 | 0.02 | 0.02 | 0.02 | 0.02 | 0.02 |
| Color change ΔE* (initial) | | | 1.1 | 2.1 | 1.0 | 1.5 | 6.0 | 1.2 | 1.2 | 1.1 | 1.5 |
| Color change ΔE* (after temperature acceleration) | | | 3.4 | 3.9 | 2.8 | 3.3 | 13.2 | 4.6 | 6.6 | 5.6 | 6.7 |

**[Table 5]**

| | Components (mass%) | | Ex. 3-1 | Ex. 3-2 | Com. Ex. 3-1 | Com. Ex. 3-2 | Com. Ex. 3-3 |
|---|---|---|---|---|---|---|---|
| First agent | Ultraviolet absorber (Example) (A) | T1 | 0.05 | | | | |
| | | T2 | | 0.05 | | | |
| | Polymerizable monomer having no acidic group (B-2) | D-2.6E | 10.00 | 10.00 | 10.00 | 10.00 | 10.00 |
| | | 3G | 5.00 | 5.00 | 5.00 | 5.00 | 5.00 |
| | | #801 | 4.00 | 4.00 | 4.00 | 4.00 | 4.00 |
| | Chemical polymerization initiator (C) | THP | 1.00 | 1.00 | 1.00 | 1.00 | 1.00 |
| | Photopolymerization initiator (E) | DABE | 0.05 | 0.05 | 0.05 | 0.05 | 0.05 |
| | Ultraviolet absorbers other than ultraviolet absorber (A) | B1 | | | | 0.05 | |
| | | B2 | | | | | 0.05 |
| | Filler (F) | SiO₂ | 1.00 | 1.00 | 1.00 | 1.00 | 1.00 |
| | | Ba1 | 28.89 | 28.89 | 28.94 | 28.89 | 28.89 |
| | Other components | BHT | 0.01 | 0.01 | 0.01 | 0.01 | 0.01 |
| Second agent | Polymerizable monomer having no acidic group (B-2) | D2.6E | 15.00 | 15.00 | 15.00 | 15.00 | 15.00 |
| | | #801 | 5.00 | 5.00 | 5.00 | 5.00 | 5.00 |
| | Chemical polymerization accelerator (D) | PyTU | 1.00 | 1.00 | 1.00 | 1.00 | 1.00 |
| | | VOAA | 0.02 | 0.02 | 0.02 | 0.02 | 0.02 |
| | Photopolymerization initiator (E) | CQ | 0.05 | 0.05 | 0.05 | 0.05 | 0.05 |
| | Filler (F) | Ba4 | 25.88 | 25.88 | 25.88 | 25.88 | 25.88 |
| | | Al₂O₃ | 3.00 | 3.00 | 3.00 | 3.00 | 3.00 |
| | Other components | BHT | 0.05 | 0.05 | 0.05 | 0.05 | 0.05 |
| Color change ΔE* (initial) | | | 0.6 | 0.7 | 5.9 | 1.0 | 2.3 |
| Color change ΔE* (after temperature acceleration) | | | 1.8 | 1.9 | 9.9 | 3.4 | 3.7 |

**[Table 6]**

| | Components (mass%) | | Ex. 4-1 | Ex. 4-2 | Com. Ex. 4-1 | Com. Ex. 4-2 | Com. Ex. 4-3 |
|---|---|---|---|---|---|---|---|
| First agent | Ultraviolet absorber (Example) (A) | T1 | 0.05 | | | | |
| | | T2 | | 0.05 | | | |
| | Polymerizable monomer having no acidic group (B-2) | UDMA | 4.70 | 4.70 | 4.70 | 4.70 | 4.70 |
| | | POBMA | 3.20 | 3.20 | 3.20 | 3.20 | 3.20 |
| | | #801 | 4.25 | 4.25 | 4.25 | 4.25 | 4.25 |
| | Chemical polymerization initiator (C) | THP | 0.50 | 0.50 | 0.50 | 0.50 | 0.50 |
| | Photopolymerization initiator (E) | BAPO | 0.03 | 0.03 | 0.03 | 0.03 | 0.03 |
| | Chemical polymerization accelerator (D) | NMDEA | 0.01 | 0.01 | 0.01 | 0.01 | 0.01 |
| | Ultraviolet absorbers other than ultraviolet absorber (A) | B1 | | | | 0.05 | |
| | | B2 | | | | | 0.05 |
| | Filler (F) | Ar380 | 0.25 | 0.25 | 0.25 | 0.25 | 0.25 |
| | | G018-090UF2.0 | 37.00 | 37.00 | 37.05 | 37.00 | 37.00 |
| | Other components | BHT | 0.01 | 0.01 | 0.01 | 0.01 | 0.01 |
| Second agent | Polymerizable monomer having no acidic group (B-2) | UDMA | 4.70 | 4.70 | 4.70 | 4.70 | 4.70 |
| | | POBMA | 3.20 | 3.20 | 3.20 | 3.20 | 3.20 |
| | | #801 | 4.25 | 4.25 | 4.25 | 4.25 | 4.25 |
| | Chemical polymerization accelerator (D) | PyTU | 0.54 | 0.54 | 0.54 | 0.54 | 0.54 |
| | | VOAA | 0.02 | 0.02 | 0.02 | 0.02 | 0.02 |
| | Photopolymerization initiator (E) | CQ | 0.03 | 0.03 | 0.03 | 0.03 | 0.03 |
| | Filler (F) | Ar380 | 0.25 | 0.25 | 0.25 | 0.25 | 0.25 |
| | | G018-090UF2.0 | 37.00 | 37.00 | 37.00 | 37.00 | 37.00 |
| | Other components | BHT | 0.01 | 0.01 | 0.01 | 0.01 | 0.01 |
| Color change ΔE* (initial) | | | 2.1 | 2.2 | 4.2 | 2.4 | 2.6 |
| Color change ΔE* (after temperature acceleration) | | | 2.8 | 2.9 | 9.1 | 3.4 | 3.7 |

As can be seen in Tables 2 to 6, the two-pack type dental compositions of the present invention, irrespective of the Example employed for preparation, exhibited little color change and superior color stability immediately after fabrication.

The two-pack type dental compositions of the present invention, irrespective of the Example employed for preparation, also exhibited little color change and superior long-term color stability even when continuously exposed to heat.

It was also confirmed that the unpolymerized layer can have a reduced thickness in the two-pack type dental compositions of the present invention, as demonstrated in Examples 1-1 to 1-8.

In contrast, the two-pack type dental compositions fabricated in Comparative Examples 1-1, 1-3, 1-5, 2-1, 3-1, and 4-1 showed large color change and lacked color stability.

Moreover, the two-pack type dental compositions of Comparative Examples, irrespective of the Comparative Example employed for preparation, exhibited large color change under continuous exposure to heat, confirming that the long-term color stability is inferior.

It is believed that these resulted from either a large shift of the peak wavelength toward the longer wavelength side of the photoabsorption spectrum due to formation of a quinoid structure in the ultraviolet absorber and the resulting extension of conjugation, or the generation of a new photoabsorption peak in the longer wavelength region as a result of coordination to a metal catalyst in the system and charge transfer between the metal and the ligand.

The two-pack type dental composition of Comparative Example 1-7, which did not contain chemical polymerization accelerator (D), did not cure, preventing evaluation of the cured product.

### INDUSTRIAL APPLICABILITY

A two-pack type dental composition of the present invention can be suitably used for restorative dental treatment. A two-pack type dental composition of the present invention is also suitable for use as a dental cement, particularly as a self-adhesive dental cement.

## Claims

1. A two-pack type dental composition comprising an ultraviolet absorber (A), a polymerizable monomer (B), a chemical polymerization initiator (C), and a chemical polymerization accelerator (D),
wherein the ultraviolet absorber (A) is a triazine-based ultraviolet absorber having a maximum absorption wavelength in a wavelength range of 310 nm or more and 400 nm or less.

2. The two-pack type dental composition according to claim 1, wherein the ultraviolet absorber (A) is a triazine-based ultraviolet absorber represented by the following general formula (1), 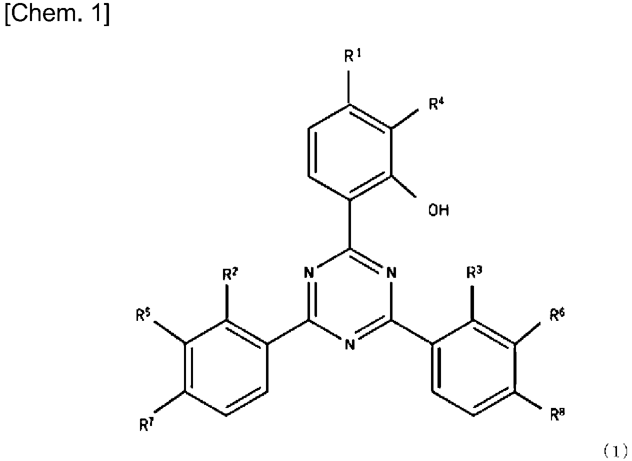 wherein R¹ represents an alkyl group or an alkoxy group, R² and R³ are the same or different from each other and each represent a hydrogen atom, a hydroxyl group, an alkyl group, an alkoxy group, or a halogen atom, R⁴, R⁵, and R⁶ are the same or different from each other and each represent a hydrogen atom or an alkyl group, and R⁷ and R⁸ are the same or different from each other and each represent a hydrogen atom, a hydroxyl group, an alkyl group, an alkoxy group, an aryl group, or a halogen atom, where the alkyl groups and alkoxy groups of R¹ to R⁸ may be optionally substituted.

3. The two-pack type dental composition according to claim 1 or 2, wherein the ultraviolet absorber (A) is 85 parts or more by mass in total 100 parts by mass of ultraviolet absorbers.

4. The two-pack type dental composition according to claim 2, wherein R¹ in the ultraviolet absorber (A) is an alkyl group or alkoxy group having 1 to 15 carbon atoms.

5. The two-pack type dental composition according to claim 4, wherein R¹ in the ultraviolet absorber (A) is a branched alkyl group or alkoxy group having 3 to 15 carbon atoms.

6. The two-pack type dental composition according to claim 5, wherein R¹ in the ultraviolet absorber (A) is a 2-ethylhexyloxy group.

7. The two-pack type dental composition according to claim 6, wherein the ultraviolet absorber (A) is bisethylhexyloxyphenol methoxyphenyl triazine and/or 2-[4,6-bis(biphenyl-4-yl)-1,3,5-triazin-2-yl]-5-[(2-ethylhexyl)oxy]phenol.

8. The two-pack type dental composition according to claim 1 or 2, wherein the chemical polymerization initiator (C) is a peroxide.

9. The two-pack type dental composition according to claim 1 or 2, wherein the chemical polymerization accelerator (D) is at least one selected from the group consisting of an aromatic sulfinic acid and a salt thereof, a sulfur-containing reducing inorganic compound, a thiourea compound, an ascorbic acid compound, a benzotriazole compound, a benzoimidazole compound, and a transition metal compound.

10. The two-pack type dental composition according to claim 1 or 2, which further comprises a photopolymerization initiator (E).

11. The two-pack type dental composition according to claim 1 or 2, which further comprises a filler (F).
